# EUROPEAN PATENT SPECIFICATION

(11) **EP 3 986 903 B1**
(45) Date of publication and mention of the grant of the patent: **10.05.2023**
(21) Application number: 20735802.9
(22) Date of filing: 16.06.2020
(51) Int. Cl.: C07F 9/6558, C07F 9/59, A61K 31/662, A61P 9/00

(54) **BIARYL DIALKYL PHOSPHINE OXIDE FPR2 AGONISTS**
BIARYLDIALKYL-PHOSPHINOXID-FPR2-AGONISTEN
AGONISTES DE FPR2 D'OXYDE DE PHOSPHINE DIALKYLIQUE DE BIARYLE

(30) Priority: 18.06.2019 US 201962862897 P
(43) Date of publication of application: 27.04.2022
(73) Proprietor: Bristol-Myers Squibb Company, Princeton, NJ 08543 (US)
(72) Inventor: SHIRUDE, Pravin Sudhakar, Bangalore Karnataka 560 099 (IN); CHATTOPADHYAY, Amit Kumar, Bangalore Karnataka 560 099 (IN); KICK, Ellen K., Princeton, New Jersey 08543 (US); WURTZ, Nicholas R., Princeton, New Jersey 08543 (US)
(74) Representative: Dehns
(86) International application number: PCT/US2020/037881
(87) International publication number: WO 2020/257161

(56) References cited:
- US-A1- 2006 074 072

## Description

### BACKGROUND OF THE INVENTION

The present invention relates to novel biaryl dialkyl phosphine oxide compounds, which are formyl peptide 2 (FPR2) receptor agonists and/or formyl peptide 1 (FPR1) receptor agonists, compositions containing them. The compounds may be used,, for example, for the treatment of atherosclerosis, heart failure, chronic obstructive pulmonary disease (COPD), and related diseases.

Formyl peptide receptor 2 (FPR2) belongs to a small group of seven-transmembrane domain, G protein-coupled receptors that are expressed in multiple human tissues including immune cells and are known to be important in host defense and inflammation. FPR2 shares significant sequence homology with FPR1 and FPR3 (Chen K, et. al., Journal of Autoimmunity 85, 2017, 64-77). Collectively, these receptors bind a number of structurally diverse agonists, including N-formyl and nonformyl peptides which act as chemo attractants and activate phagocytes. The endogenous peptide Annexin A1 and its N-terminal fragments are examples of ligands that bind human FPR1 and FPR2. Fatty acids such as eicosanoid, lipoxin A4, which belongs to a class of small pro-resolution mediators (SPMs), has also been identified as an agonist for FPR2 (Ye RD., et al., Pharmacol. Rev., 2009, 61, 119-61).

Endogenous FPR2 pro-resolution ligands, such as lipoxin A₄ and Annexin A1, have been reported to trigger a wide array of cytoplasmatic cascades such as Gi coupling, Ca²⁺ mobilization and β-arrestin recruitment. (Cattaneo, F, et. al., Int J Mol Sci. 2013 April; 14(4): 7193-7230). FPR2 regulates both innate and adaptive immune systems including neutrophils, macrophages, T-, and B-cells. In neutrophils, FPR2 ligands modulate movement, cytotoxicity and life span. In macrophages, agonism of FPR2 prevents apoptosis and enhances efferocytosis. (Chandrasekharan JA, Sharma-Walia N,. J. Inflamm. Res., 2015, 8, 181-92). The initiation of resolution of inflammation by FPR2 agonism is responsible for enhancing anti-fibrotic wound healing and returning of the injured tissue to homeostasis (Romano M., et al., Eur. J. Pharmacol., 2015, 5, 49-63).

Chronic inflammation is part of the pathway of pathogenesis of many human diseases and stimulation of resolution pathways with FPR2 agonists may have both protective and reparative effects. Ischaemia-reperfusion (I/R) injury is a common feature of several diseases associated with high morbidity and mortality, such as myocardial infarction and stroke. Non-productive wound healing associated with cardiomyocyte death and pathological remodeling resulting from ischemia-reperfusion injury leads to scar formation, fibrosis, and progressive loss of heart function. FPR2 modulation is proposed to enhance myocardial wound healing post injury and diminish adverse myocardial remodeling (Kain V., et al., J. Mol. Cell. Cardiol., 2015, 84, 24-35). In addition, FPR2 pro-resolution agonists, in the central nervous system, may be useful therapeutics for the treatment of a variety of clinical I/R conditions, including stroke in brain (Gavins FN., Trends Pharmacol. Sci., 2010, 31, 266-76) and I/R induced spinal cord injury (Liu ZQ ., et al., Int. J. Clin. Exp. Med., 2015, 8, 12826-33).

In addition to beneficial effects of targeting the FPR2 receptor with novel pro-resolution agonists for treatment of I/R induced injury therapeutic, utility of these ligands can also be applied to other diseases. In the cardiovascular system both the FPR2 receptor and its pro-resolution agonists were found to be responsible for atherogenic-plaque stabilization and healing (Petri MH., et al., Cardiovasc. Res., 2015, 105, 65-74; and Fredman G., et al., Sci. Trans. Med., 2015, 7(275);275ra20). FPR2 agonists also have been shown to be beneficial in preclinical models of chronic inflammatory human diseases, including: infectious diseases, psoriasis, dermatitis, inflammatory bowel syndrome, Crohn's disease, occular inflammation, sepsis, pain, metabolic/diabetes diseases, cancer, COPD, asthma and allergic diseases, cystic fibrosis, acute lung injury and fibrosis, rheumatoid arthritis and other joint diseases, Alzheimer's disease, kidney fibrosis, and organ transplantation (Romano M., et al., Eur. J. Pharmacol., 2015, 5, 49-63, Perrett, M., et al., Trends in Pharm. Sci., 2015, 36, 737-755). US2006/074072 A1 discloses compounds useful for the treatment of myocardial infarction.

### SUMMARY OF THE INVENTION

The present invention provides novel biaryl dialkyl phosphine oxide compounds, and their analogues thereof, which are useful as FPR2 agonists, including stereoisomers, tautomers, pharmaceutically acceptable salts, or solvates thereof.

The present invention also provides pharmaceutical compositions comprising a pharmaceutically acceptable carrier and at least one of the compounds of the present invention or stereoisomers, tautomers, pharmaceutically acceptable salts, or solvates thereof.

The compounds of the invention may be used in therapy.

The compounds of the invention may be used in the treatment and/or prophylaxis of multiple diseases or disorders associated with FPR2, such as inflammatory diseases, heart diseases, chronic airway diseases, cancers, septicemia, allergic symptoms, HIV retrovirus infection, circulatory disorders, neuroinflammation, nervous disorders, pains, prion diseases, amyloidosis, and immune disorders. The heart diseases are selected from the group consisting of angina pectoris, unstable angina, myocardial infarction, acute coronary disease, cardiac iatrogenic damage, and heart failure including, but not limited to, acute heart failure, chronic heart failure of ischemic and non-ischemic origin, systolic heart failure, diastolic heart failure, heart failure with reduced ejection fraction (HF_{R}EF), and heart failure with preserved ejection fraction (HFpEF).

The compounds of the invention can be used alone, in combination with other compounds of the present invention, or in combination with one or more other agent(s).

Other features and advantages of the invention will be apparent from the following detailed description and claims.

### DESCRIPTION OF THE INVENTION

The invention encompasses compounds of Formula (I), which are formyl peptide 2 (FPR2) receptor agonists and/or formyl peptide 1 (FPR1) receptor agonists, compositions containing them, and the compounds or compositions for use in the treatment of atherosclerosis, heart failure, chronic obstructive pulmonary disease (COPD), and related diseases, for example.

One aspect of the invention is a compound of Formula (I): 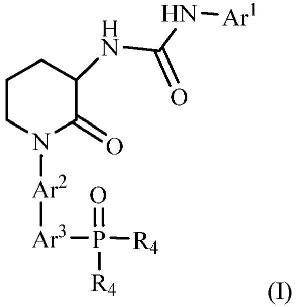 or a stereoisomer, a tautomer, or a pharmaceutically acceptable salt thereof, wherein:
Ar¹ is phenyl substituted with 1-2 R^{1a} and 1-2 R^{1b} or 6-membered heteroaryl with 1-3 nitrogen atoms and substituted with 1-2 R^{1a} and 1-2 R^{1b};
Ar² is phenyl substituted with 1-4 R² or 6-membered heteroaryl with 1-3 nitrogen atoms and substituted with 1-4 R²;
Ar³ is phenyl substituted with 0-4 R³ or pyridinyl substituted with 0-4 R³;
R^{1a} is hydrogen or halo;
R^{1b} is halo, haloalkyl, alkoxy, or haloalkoxy;
R² is hydrogen, cyano, halo, alkyl, hydroxyalkyl, haloalkyl, cycloalkyl, alkoxy, or haloalkoxy;
R³ is cyano, halo, alkyl, alkoxy, hydroxyalkyl, alkoxyalkyl, or haloalkyl; and
R⁴ is alkyl or alkoxy.

Another aspect of the invention is a compound of Formula (II): 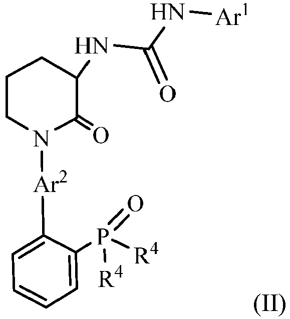 or a stereoisomer, a tautomer, or a pharmaceutically acceptable salt thereof, wherein:
Ar¹ is phenyl substituted with 1 R^{1a} and 1-2 R^{1b} or 6-membered heteroaryl with 1-2 nitrogen atoms and substituted with 1R^{1a} and 1-2 R^{1b};
Ar² is phenyl substituted with 1-3 R² or pyridinyl substituted with 1-4 R²;
R^{1a} is hydrogen or halo;
R^{1b} is halo, C₁₋₄ haloalkyl, C₁₋₄ alkoxy, or C₁₋₄ haloalkoxy;
R² is hydrogen, halo, C₁₋₄ alkyl, C₁₋₄ hydroxyalkyl, C₁₋₄ haloalkyl, C₃₋₆ cycloalkyl, C₁₋₄ alkoxy, or C₁₋₄ haloalkoxy; and
R⁴ is C₁₋₃ alkyl.

Another aspect of the invention is a compound of Formula (III): 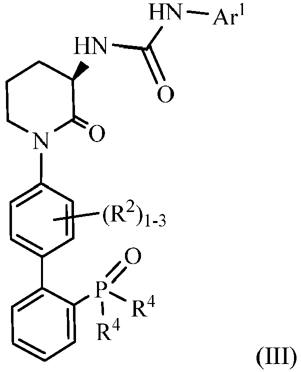 or a stereoisomer, a tautomer, or a pharmaceutically acceptable salt thereof, wherein:
Ar¹ is phenyl substituted with 1R^{1a} and 1-2 R^{1b}, pyridinyl substituted with 1 R^{1a} and 1-2 R^{1b}, or pyrazinyl substituted with 1R^{1a} and 1-2 R^{1b};
R^{1a} is hydrogen or halo;
R^{1b} is halo, C₁₋₄ haloalkyl, C₁₋₄ alkoxy, or C₁₋₄ haloalkoxy;
R² is hydrogen, halo, C₁₋₄ alkyl, C₁₋₄ hydroxyalkyl, C₁₋₄ haloalkyl, C₃₋₆ cycloalkyl, C₁₋₄ alkoxy, or C₁₋₄ haloalkoxy; and
R⁴ is C₁₋₂ alkyl.

Another aspect of the invention is a compound of Formula (IV): 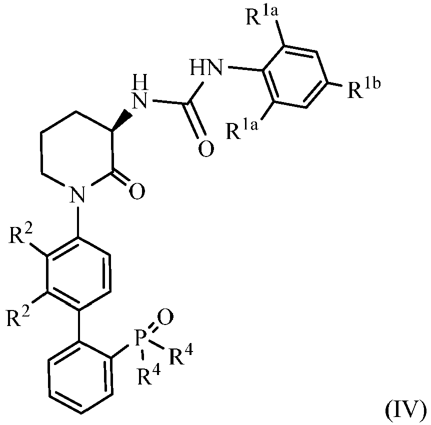 or a stereoisomer, a tautomer, or a pharmaceutically acceptable salt thereof, wherein:
R^{1a} is hydrogen or F;
R^{1b} is halo, C₁₋₂ haloalkyl, or C₁₋₂ alkoxy;
R² is hydrogen, halo, C₁₋₃ alkyl, C₁₋₃ haloalkyl, or C₃₋₆ cycloalkyl; and
R⁴ is methyl or ethyl.

Another aspect of the invention is a compound of Formula (IV) or (VI), or a stereoisomer, a tautomer, or a pharmaceutically acceptable salt thereof, wherein:
R^{1a} is hydrogen or F;
R^{1b} is F, Cl, or CF₃; and
R² is hydrogen, F, Cl, or cyclopropyl; and
R⁴ is methyl.

Another aspect of the invention is a compound of Formula (V): 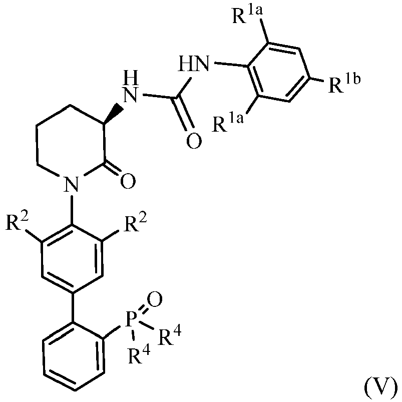 or a stereoisomer, a tautomer, or a pharmaceutically acceptable salt thereof, wherein:
R^{1a} is hydrogen or F;
R^{1b} is halo, C₁₋₂ haloalkyl, or C₁₋₂ alkoxy;
R² is halo; and
R⁴ is methyl or ethyl.

Another aspect of the invention is a compound of Formula (VI): 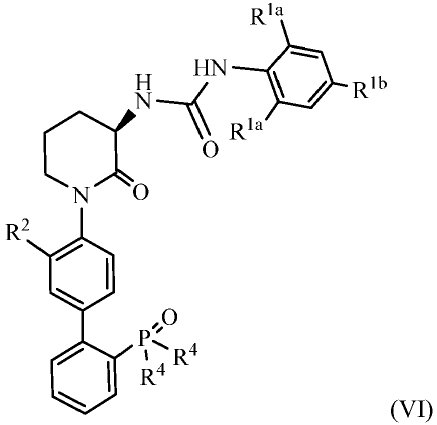 or a stereoisomer, a tautomer, or a pharmaceutically acceptable salt thereof, wherein:
R^{1a} is hydrogen or F;
R^{1b} is halo, C₁₋₂ haloalkyl, or C₁₋₂ alkoxy;
R² is halo, C₁₋₃ alkyl, C₁₋₃ haloalkyl, or C₃₋₆ cycloalkyl; and
R⁴ is methyl or ethyl.

Another aspect of the invention is a compound of Formula (VII): 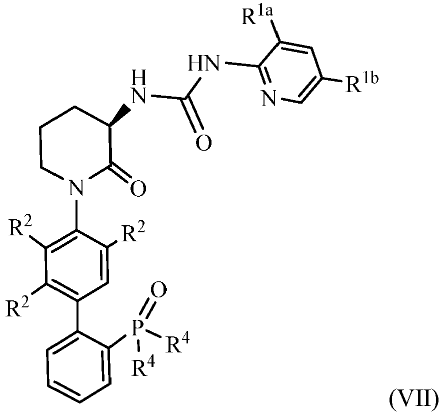 or a stereoisomer, a tautomer, or a pharmaceutically acceptable salt thereof, wherein:
R^{1a} is hydrogen or halo;
R^{1b} is halo, C₁₋₂ haloalkyl, or C₁₋₂ alkoxy;
R² is hydrogen, halo, C₁₋₃ alkyl, or C₃₋₆ cycloalkyl; and
R⁴ is C₁₋₂ alkyl.

Another aspect of the invention is a compound of Formula (VIII): 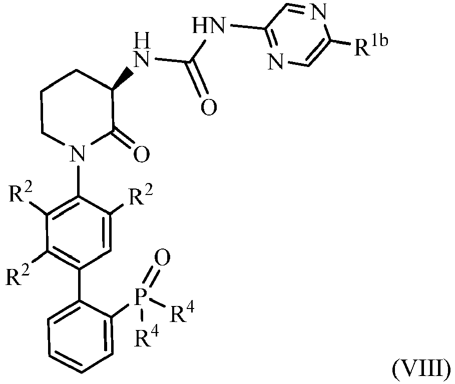 or a stereoisomer, a tautomer, or a pharmaceutically acceptable salt thereof, wherein:
R^{1b} is halo, C₁₋₂ haloalkyl, or C₁₋₂ alkoxy;
R² is hydrogen, halo, C₁₋₃ alkyl, or C₃₋₆ cycloalkyl; and
R⁴ is C₁₋₂ alkyl.

Another aspect of the invention is a compound of Formula (IX): 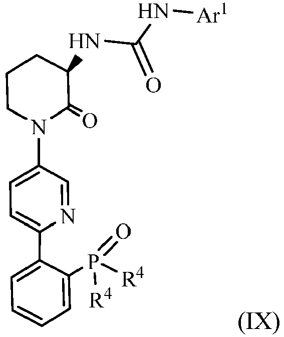 or a stereoisomer, a tautomer, or a pharmaceutically acceptable salt thereof, wherein:
Ar¹ is phenyl substituted with 1R^{1a} and 1-2 R^{1b}, pyridinyl substituted with 1 R^{1a} and 1-2 R^{1b}, or pyrazinyl substituted with 1R^{1a} and 1-2 R^{1b}.
R^{1a} is hydrogen or halo;
R^{1b} is halo, C₁₋₄ haloalkyl, C₁₋₄ alkoxy, or C₁₋₄ haloalkoxy; and
R⁴ is C₁₋₂ alkyl.

Another aspect of the invention is a compound selected from the group consisting of: 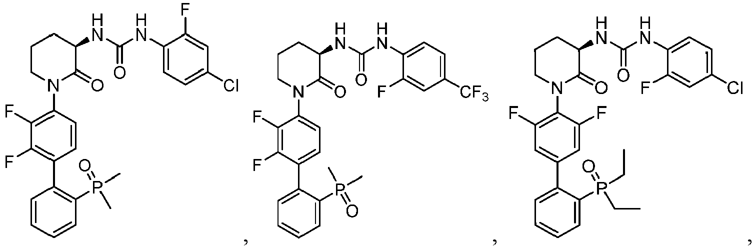 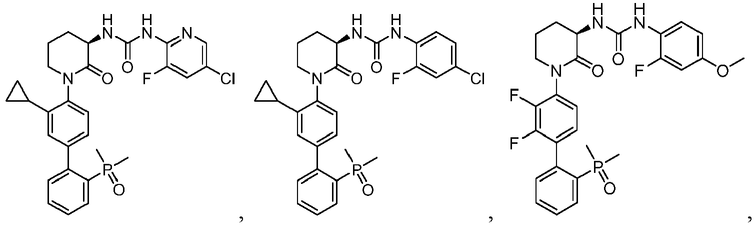 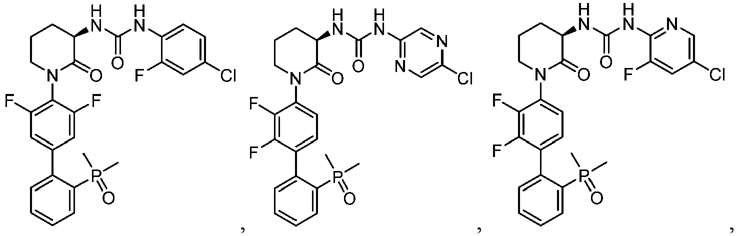 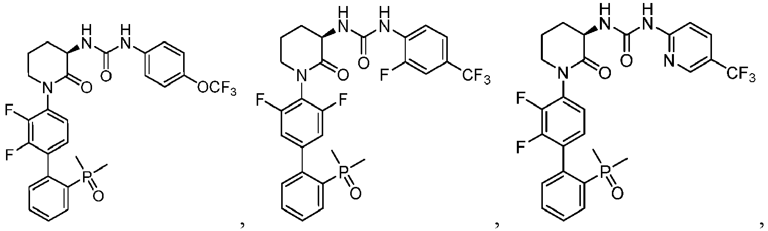 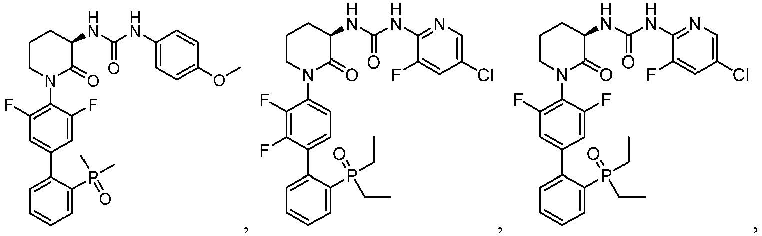 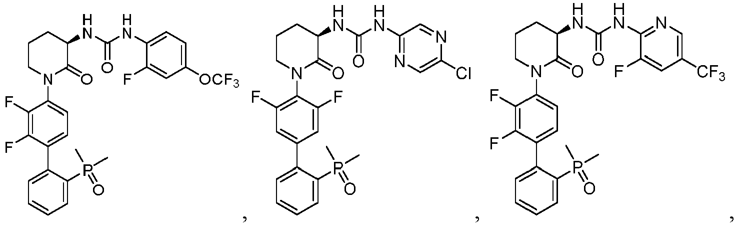 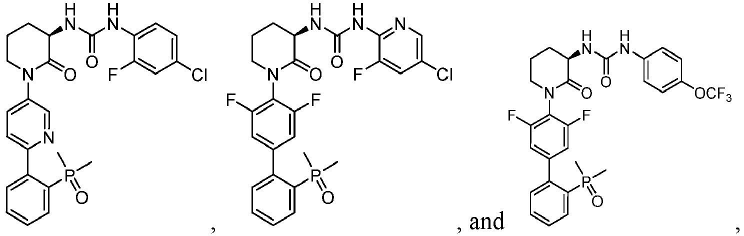 or a stereoisomer, a tautomer, or a pharmaceutically acceptable salt thereof.

For a compound of Formula (I), (II), (III), (IV), (V), (VI), (VII), (VIII), or (IX), the scope of any instance of a variable substituent, including Ar¹, Ar², Ar³, R^{1a}, R^{1b}, R², R³, and R⁴ can be used independently with the scope of any other instance of a variable substituent. As such, the invention includes combinations of the different aspects.

Unless specified otherwise, these terms have the following meanings. "Alkyl" means a straight or branched alkyl group composed of 1 to 6 carbons. "Alkenyl" means a straight or branched alkyl group composed of 2 to 6 carbons with at least one double bond. "Alkynyl" means a straight or branched alkyl group composed of 2 to 6 carbons with at least one triple bond. "Cycloalkyl" means a monocyclic ring system composed of 3 to 7 carbons. Terms with a hydrocarbon moiety (e.g. alkoxy) include straight and branched isomers for the hydrocarbon portion. "Halo" includes fluoro, chloro, bromo, and iodo. "Haloalkyl" and "haloalkoxy" include all halogenated isomers from monohalo to perhalo. "Aryl" means a monocyclic or bicyclic aromatic hydrocarbon groups having 6 to 12 carbon atoms, or a bicyclic fused ring system wherein one or both of the rings is aromatic. Bicyclic fused ring systems consist of a phenyl group fused to a four- to seven-membered aromatic or non-aromatic carbocyclic ring. Representative examples of aryl groups include but are not limited to phenyl, indanyl, indenyl, naphthyl, and tetrahydronaphthyl. "Heteroaryl" means a 5 to 7 membered monocyclic or 8 to 11 membered bicyclic aromatic ring system with 1-5 heteroatoms independently selected from nitrogen, oxygen, and sulfur. Where a bonding attachment location is not specified, the bonding may be attached at any appropriate location as understood by practitioners in the art. Combinations of substituents and bonding patterns are only those that result in stable compounds as understood by practitioners in the art. Parenthetic and multiparenthetic terms are intended to clarify bonding relationships to those skilled in the art. For example, a term such as ((R)alkyl) means an alkyl substituent further substituted with the substituent R.

The invention includes all pharmaceutically acceptable salt forms of the compounds. Pharmaceutically acceptable salts are those in which the counter ions do not contribute significantly to the physiological activity or toxicity of the compounds and as such function as pharmacological equivalents. These salts can be made according to common organic techniques employing commercially available reagents. Some anionic salt forms include acetate, acistrate, besylate, bromide, chloride, citrate, fumarate, glucouronate, hydrobromide, hydrochloride, hydroiodide, iodide, lactate, maleate, mesylate, nitrate, pamoate, phosphate, succinate, sulfate, tartrate, tosylate, and xinofoate. Some cationic salt forms include ammonium, aluminum, benzathine, bismuth, calcium, choline, diethylamine, diethanolamine, lithium, magnesium, meglumine, 4-phenylcyclohexylamine, piperazine, potassium, sodium, tromethamine, and zinc.

Some of the compounds of the invention exist in stereoisomeric forms including the structure below with the indicated carbon. The invention includes all stereoisomeric forms of the compounds including enantiomers and diastereomers. Methods of making and separating stereoisomers are known in the art. The invention includes all tautomeric forms of the compounds. The invention includes atropisomers and rotational isomers.

The invention is intended to include all isotopes of atoms occurring in the compounds. Isotopes include those atoms having the same atomic number but different mass numbers. By way of general example and without limitation, isotopes of hydrogen include deuterium and tritium. Isotopes of carbon include ¹³C and ¹⁴C. Isotopically-labeled compounds of the invention can generally be prepared by conventional techniques known to those skilled in the art or by processes analogous to those described herein, using an appropriate isotopically-labeled reagent in place of the non-labeled reagent otherwise employed. Such compounds may have a variety of potential uses, for example as standards and reagents in determining biological activity. In the case of stable isotopes, such compounds may have the potential to favorably modify biological, pharmacological, or pharmacokinetic properties.

### BIOLOGICAL METHODS

N-formyl peptide receptors (FPRs) are a family of chemo attractant receptors that facilitate leukocyte response during inflammation. FPRs belong to the seven-transmembrane G protein-coupled receptor superfamily and are linked to inhibitory G-proteins (Gi). Three family members (FPR1, FPR2 and FPR3) have been identified in humans and are predominantly found in myeloid cells with varied distribution and have also been reported in multiple organs and tissues. After agonist binding, the FPRs activate a multitude of physiological pathways, such as intra cellular signaling transduction, Ca²⁺ mobilization and transcription. The family interacts with a diverse set of ligands that includes proteins, polypeptides and fatty acid metabolites which activate both pro-inflammatory and pro-resolution downstream responses. FPR2 and FPR1 Cyclic Adenosine Monophosphate (cAMP) Assays were used to measure the activity of the compounds in this patent.

FPR2 and FPR1 Cyclic Adenosine Monophosphate (cAMP) Assays. A mixture of forskolin (5 µM final for FPR2 or 10 µM final for FPR1) and IBMX (200 µM final) were added to 384-well Proxiplates (Perkin-Elmer) pre-dotted with test compounds in DMSO (1% final) at final concentrations in the range of 0.020 nM to 100 µM. Chinese Hamster Ovary cells (CHO) overexpressing human FPR1 or human FPR2 receptors were cultured in F-12 (Ham's) medium supplemented with 10% qualified FBS, 250 µg/mL zeocin and 300 µg/mL hygromycin (Life Technologies). Reactions were initiated by adding 2,000 human FPR2 cells per well or 4,000 human FPR1 cells per well in Dulbecco's PBS (with calcium and magnesium) (Life Technologies) supplemented with 0.1% BSA (Perkin-Elmer). The reaction mixtures were incubated for 30 min at rt. The level of intracellular cAMP was determined using the HTRF HiRange cAMP assay reagent kit (Cisbio) according to manufacturer's instruction. Solutions of cryptate conjugated anti-cAMP and d2 flurorophore-labelled cAMP were made in a supplied lysis buffer separately. Upon completion of the reaction, the cells were lysed with equal volume of the d2-cAMP solution and anti-cAMP solution. After a 1-h rt incubation, time-resolved fluorescence intensity was measured using the Envision (Perkin-Elmer) at 400 nm excitation and dual emission at 590 nm and 665 nm. A calibration curve was constructed with an external cAMP standard at concentrations ranging from 1 µM to 0.1 pM by plotting the fluorescent intensity ratio from 665 nm emission to the intensity from the 590 nm emission against cAMP concentrations. The potency and activity of a compound to inhibit cAMP production was then determined by fitting to a 4-parametric logistic equation from a plot of cAMP level versus compound concentrations.

The examples disclosed below were tested in the FPR2 and FPR1 cAMP assay described above and found having FPR2 and/or FPR1 agonist activity. Table 1 below lists EC₅₀ values in the FPR2 and FPR1 cAMP assays measured for the following examples.

**Table 1**

| Example | hFPR2 cAMP2 EC₅₀ (µM) | hFPR1 cAMP EC₅₀ (µM) |
|---|---|---|
| 1 | 0.00012 | 0.11 |
| 2 | 0.00018 | 0.30 |
| 3 | 0.00017 | 0.42 |
| 4 | 0.00063 | 0.0071 |
| 5 | 0.00076 | 0.017 |
| 6 | 0.00086 | 0.43 |
| 7 | 0.00079 | 2.6 |
| 8 | 0.0015 | 1.1 |
| 9 | 0.0022 | 1.4 |
| 10 | 0.0014 | 0.37 |
| 11 | 0.0026 | 1.7 |
| 12 | 0.0027 | 1.3 |
| 13 | 0.0056 | 2.4 |
| 14 | 0.0060 | 1.5 |
| 15 | 0.0083 | 7.0 |
| 16 | 0.0084 | 1.8 |
| 17 | 0.0086 | >10 |
| 18 | 0.0116 | >10 |
| 19 | 0.0167 | 0.14 |
| 20 | 0.0432 | >10 |
| 21 | 0.0221 | >10 |

### PHARMACEUTICAL COMPOSITIONS AND METHODS OF USE

The compounds of the present invention may be administered to mammals, preferably humans, for the treatment of a variety of conditions and disorders associated with the FPR2 receptor such as Behcet's disease, Sweet disease, systemic lupus erythematosus (SLE), Wegener's granulomatosis, virus infection, diabetes, amputations, cancers, bacterial infection, physical external injuries, physical disorders including exposure to radiation, vasoconstriction, anaphylactic reactions, allergic reactions, rhinitis, shocks (endotoxic, hemorrhagic, traumatic, splanchnic ischemia, and circulatory shocks), rheumatoid arthritis, gout, psoriasis, benign prostatic hyperplasia, myocardial ischemia, myocardial infarction, heart failure, brain injuries, pulmonary diseases, COPD, COAD, COLD, acute lung injury, acute respiratory distress syndrome, chronic bronchitis, pulmonary emphysema, asthma (allergic asthma and non-allergic asthma), cystic fibrosis, kidney fibrosis, nephropathy, renal glomerular diseases, ulcerative colitis, IBD, Crohn's disease, periodontitis, pains, Alzheimer's disease, AIDS, uveitic glaucoma, conjunctivitis, Sjoegren's syndrome, rhinitis, atherosclerosis, neuroinflammatory diseases including multiple sclerosis, stroke, sepsis, and the like.

Unless otherwise specified, the following terms have the stated meanings. The term "subject" refers to any human or other mammalian species that could potentially benefit from treatment with a FPR2 and/or FPR1 agonist as understood by practioners in this field. Some subjects include human beings of any age with risk factors for cardiovascular disease. Common risk factors include age, sex, weight, family history, sleep apnea, alcohol or tobacco use, physical inactivity arrthymia or signs of insulin resistance such as acanthosis nigricans, hypertension, dyslipidemia, or polycystic ovary syndrome (PCOS). The term "patient" means a person suitable for therapy as determined by practitioners in the field. "Treating" or "treatment" cover the treatment of a patient or subject as understood by practitioners in this field. "Preventing" or "prevention" cover the preventive treatment (i.e., prophylaxis and/or risk reduction) of a subclinical disease-state in a patient or subject aimed at reducing the probability of the occurrence of a clinical disease-state as understood by practitioners in this field. Patients are selected for preventative therapy based on factors that are known to increase risk of suffering a clinical disease state compared to the general population. "Therapeutically effective amount" means an amount of a compound that is effective as understood by practitioners in this field.

Another aspect of the invention are pharmaceutical compositions comprising a therapeutically effective amount of a compound of Formulae (I)-(IX) in combination with a pharmaceutical carrier.

Another aspect of the invention are pharmaceutical compositions comprising a therapeutically effective amount of a compound of Formulae (I)-(IX) in combination with at least one other therapeutic agent and a pharmaceutical carrier.

"Pharmaceutical composition" means a composition comprising a compound of the invention in combination with at least one additional pharmaceutically acceptable carrier. A "pharmaceutically acceptable carrier" refers to media generally accepted in the art for the delivery of biologically active agents to animals, in particular, mammals, including, i.e., adjuvant, excipient or vehicle, such as diluents, preserving agents, fillers, flow regulating agents, disintegrating agents, wetting agents, emulsifying agents, suspending agents, sweetening agents, flavoring agents, perfuming agents, anti-bacterial agents, anti-fungal agents, lubricating agents and dispensing agents, depending on the nature of the mode of administration and dosage forms.

Pharmaceutically acceptable carriers are formulated according to a number of factors well within the purview of those of ordinary skill in the art. These include, without limitation: the type and nature of the active agent being formulated; the subject to which the agent-containing composition is to be administered; the intended route of administration of the composition; and the therapeutic indication being targeted. Pharmaceutically acceptable carriers include both aqueous and non-aqueous liquid media, as well as a variety of solid and semi-solid dosage forms. Such carriers can include a number of different ingredients and additives in addition to the active agent, such additional ingredients being included in the formulation for a variety of reasons, e.g., stabilization of the active agent, binders, etc., well known to those of ordinary skill in the art. Descriptions of suitable pharmaceutically acceptable carriers, and factors involved in their selection, are found in a variety of readily available sources such as, for example, Allen, L.V., Jr. et al., Remington: The Science and Practice of Pharmacy (2 Volumes), 22nd Edition, Pharmaceutical Press (2012).

Particularly when provided as a single dosage unit, the potential exists for a chemical interaction between the combined active ingredients. For this reason, when the compound of the present invention and a second therapeutic agent are combined in a single dosage unit they are formulated such that although the active ingredients are combined in a single dosage unit, the physical contact between the active ingredients is minimized (that is, reduced). For example, one active ingredient may be enteric coated. By enteric coating one of the active ingredients, it is possible not only to minimize the contact between the combined active ingredients, but also, it is possible to control the release of one of these components in the gastrointestinal tract such that one of these components is not released in the stomach but rather is released in the intestines. One of the active ingredients may also be coated with a material that affects a sustained-release throughout the gastrointestinal tract and also serves to minimize physical contact between the combined active ingredients. Furthermore, the sustained-released component can be additionally enteric coated such that the release of this component occurs only in the intestine. Still another approach would involve the formulation of a combination product in which the one component is coated with a sustained and/or enteric release polymer, and the other component is also coated with a polymer such as a low viscosity grade of hydroxypropyl methylcellulose (HPMC) or other appropriate materials as known in the art, in order to further separate the active components. The polymer coating serves to form an additional barrier to interaction with the other component.

Compounds of the present invention may be used in a method for treating heart disease comprising administering a therapeutically effective amount of a compound of Formulae (I)-(IX) to a patient.

Compounds of the present invention may be used in a method for treating heart disease wherein the heart disease is selected from the group consisting of angina pectoris, unstable angina, myocardial infarction, heart failure, acute coronary disease, acute heart failure, chronic heart failure, and cardiac iatrogenic damage.

It will be understood that treatment or prophylaxis of heart failure may involve treatment or prophylaxis of a cardiovascular event as well. Treatment or prophylaxis as referred to herein may refer to treatment or prophylaxis of certain negative symptoms or conditions associated with or arising as a result of a cardiovascular event. By way of example, treatment or prophylaxis may involve reducing or preventing negative changes in fractional shortening, heart weight, lung weight, myocyte cross sectional area, pressure overload induced cardiac fibrosis, stress induced cellular senescence, and/or cardiac hypertrophy properties, or any combination thereof, associated with or arising as a result of a cardiovascular event. Treatment may be administered in preparation for or in response to a cardiovascular event to alleviate negative effects. Prevention may involve a pro-active or prophylactic type of treatment to prevent the cardiovascular event or to reduce the onset of negative effects of a cardiovascular event.

Compounds of Formulae (I)-(IX) or a pharmaceutically acceptable salt thereof may be used for the preparation of a pharmaceutical composition for the treatment or prophylaxis of heart failure, for example, heart failure results from hypertension, an ischemic heart disease, a non-ischemic heart disease, exposure to a cardiotoxic compound, myocarditis, Kawasaki's disease, Type I and Type II diabetes, thyroid disease, viral infection, gingivitis, drug abuse, alcohol abuse, pericarditis, atherosclerosis, vascular disease, hypertrophic cardiomyopathy, dilated cardiomyopathy, myocardial infarction, atrial fibrosis, left ventricular systolic dysfunction, left ventricular diastolic dysfunction, coronary bypass surgery, pacemaker implantation surgery, starvation, an eating disorder, muscular dystrophies, and a genetic defect. The heart failure to be treated may be diastolic heart failure, heart failure with reduced ejection fraction (HF_{R}EF), heart failure with preserved ejection fraction (HF_{P}EF), acute heart failure, and chronic heart failure of ischemic and non-ischemic origin.

Compounds of Formulae (I)-(IX) may be used in a method to treat systolic and/or diastolic dysfunction, wherein the compound is administered in a therapeutically effective amount to increase the ability of the cardiac muscle cells to contract and relax thereby increasing the filling and emptying of both the right and left ventricles, preferably, the left ventricle.

Compounds of Formulae (I)-(IX) may be used in a method to treat heart failure wherein the compound is administered in a therapeutically effective amount to increase ejection fraction in the left ventricle.

Compounds of Formulae (I)-(IX) may be used in a method to treat heart failure wherein the compound is administered in a therapeutically effective amount to reduce fibrosis in heart tissue.

Compounds of the present invention may be used in a method for treating heart disease wherein the treatment is post myocardial infarction.

Compounds of the present invention may be used in a method for treating heart disease comprising administering a therapeutically effective amount of a compound of Formulae (I)-(IX) to a patient in conjuction with other therapeutic agents.

The compounds of this invention may be administered by any suitable means, for example, orally, such as tablets, capsules (each of which includes sustained release or timed release formulations), pills, powders, granules, elixirs, tinctures, suspensions (including nanosuspensions, microsuspensions, spray-dried dispersions), syrups, and emulsions; sublingually; bucally; parenterally, such as by subcutaneous, intravenous, intramuscular, or intrasternal injection, or infusion techniques (e.g., as sterile injectable aqueous or non-aqueous solutions or suspensions); nasally, including administration to the nasal membranes, such as by inhalation spray; topically, such as in the form of a cream or ointment; or rectally such as in the form of suppositories. They may be administered alone, or may be administered with a pharmaceutical carrier selected on the basis of the chosen route of administration and standard pharmaceutical practice.

The dosage regimen for the compounds of the present invention will, of course, vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired.

By way of general guidance, the daily oral dosage of each active ingredient, when used for the indicated effects, will range between about 0.01 to about 5000 mg per day, preferably between about 0.1 to about 1000 mg per day, and most preferably between about 0.1 to about 250 mg per day. Intravenously, the most preferred doses will range from about 0.01 to about 10 mg/kg/minute during a constant rate infusion. Compounds of this invention may be administered in a single daily dose, or the total daily dosage may be administered in divided doses of two, three, or four times daily.

Dosage forms (pharmaceutical compositions) suitable for administration may contain from about 1 milligram to about 2000 milligrams of active ingredient per dosage unit. In these pharmaceutical compositions the active ingredient will ordinarily be present in an amount of about 0.1-95% by weight based on the total weight of the composition. A typical capsule for oral administration contains at least one of the compounds of the present invention (250 mg), lactose (75 mg), and magnesium stearate (15 mg). The mixture is passed through a 60 mesh sieve and packed into a No. 1 gelatin capsule. A typical injectable preparation is produced by aseptically placing at least one of the compounds of the present invention (250 mg) into a vial, aseptically freeze-drying and sealing. For use, the contents of the vial are mixed with 2 mL of physiological saline, to produce an injectable preparation.

The compounds of the present invention may be employed in combination with other suitable therapeutic agents useful in the treatment of the aforementioned diseases or disorders including: anti-atherosclerotic agents, anti-dyslipidemic agents, anti-diabetic agents, anti-hyperglycemic agents, anti-hyperinsulinemic agents, anti-thrombotic agents, anti-retinopathic agents, anti-neuropathic agents, anti-nephropathic agents, anti-ischemic agents, anti-hypertensive agents, anti-obesity agents, anti-hyperlipidemic agents, anti-hypertriglyceridemic agents, anti-hypercholesterolemic agents, anti-restenotic agents, anti-pancreatic agents, lipid lowering agents, anorectic agents, memory enhancing agents, anti-dementia agents, cognition promoting agents, appetite suppressants, agents for treating heart failure, agents for treating peripheral arterial disease, agents for treating malignant tumors, and anti-inflammatory agents.

The compounds of the invention may be used with at least one of the following heart failure agents selected from loop diuretics, Angiotensin converting enzyme (ACE) inhibitors, Angiotensin II receptor blockers (ARBs), angiotensin receptor-neprilysin inhibitors (ARNI), beta blockers, mineralocorticoid receptor antagonists, nitroxyl donors, RXFP1 agonists, APJ agonists and cardiotonic agents. These agents include, but are not limited to furosemide, bumetanide, torsemide, sacubitrial-valsartan, thiazide diruetics, captopril, enalapril, lisinopril, carvedilol, metopolol, bisoprolol, serelaxin, spironolactone, eplerenone, ivabradine, candesartan, eprosartan, irbestarain, losartan, olmesartan, telmisartan, and valsartan.

The compounds of the present invention may be employed in combination with at least one of the following therapeutic agents in treating atherosclerosis: anti-hyperlipidemic agents, plasma HDL-raising agents, anti-hypercholesterolemic agents, cholesterol biosynthesis inhibitors (such as HMG CoA reductase inhibitors), LXR agonist, probucol, raloxifene, nicotinic acid, niacinamide, cholesterol absorption inhibitors, bile acid sequestrants (such as anion exchange resins, or quaternary amines (e.g., cholestyramine or colestipol)), low density lipoprotein receptor inducers, clofibrate, fenofibrate, benzofibrate, cipofibrate, gemfibrizol, vitamin B₆, vitamin B₁₂, anti-oxidant vitamins, β-blockers, anti-diabetes agents, angiotensin II antagonists, angiotensin converting enzyme inhibitors, platelet aggregation inhibitors, fibrinogen receptor antagonists, aspirin and fibric acid derivatives.

The compounds of the present invention may be employed in combination at least one of the following therapeutic agents in treating cholesterol biosynthesis inhibitor, particularly an HMG-CoA reductase inhibitor. Examples of suitable HMG-CoA reductase inhibitors include, but are not limited to, lovastatin, simvastatin, pravastatin, fluvastatin, atorvastatin, and rosuvastatin.

The compounds of the invention may be used in combination with at least one of the following anti-diabetic agents depending on the desired target therapy. Studies indicate that diabetes and hyperlipidemia modulation can be further improved by the addition of a second agent to the therapeutic regimen. Examples of anti-diabetic agents include, but are not limited to, sulfonylureas (such as chlorpropamide, tolbutamide, acetohexamide, tolazamide, glyburide, gliclazide, glynase, glimepiride, and glipizide), biguanides (such as metformin), thiazolidinediones (such as ciglitazone, pioglitazone, troglitazone, and rosiglitazone), and related insulin sensitizers, such as selective and non-selective activators of PPARα, PPARβ and PPARγ; dehydroepiandrosterone (also referred to as DHEA or its conjugated sulphate ester, DHEA-SO₄); anti-glucocorticoids; TNFα inhibitors; dipeptidyl peptidase IV (DPP4) inhibitor (such as sitagliptin, saxagliptin), GLP-1 agonists or analogs (such as exenatide), α-glucosidase inhibitors (such as acarbose, miglitol, and voglibose), pramlintide (a synthetic analog of the human hormone amylin), other insulin secretagogues (such as repaglinide, gliquidone, and nateglinide), insulin, as well as the therapeutic agents discussed above for treating atherosclerosis.

The compounds of the invention may be used in combination with at least one of the following anti-obesity agents selected from phenylpropanolamine, phentermine, diethylpropion, mazindol, fenfluramine, dexfenfluramine, phentiramine, β₃-adrenoreceptor agonist agents; sibutramine, gastrointestinal lipase inhibitors (such as orlistat), and leptins. Other agents used in treating obesity or obesity-related disorders include neuropeptide Y, enterostatin, cholecytokinin, bombesin, amylin, histamine H₃ receptors, dopamine D₂ receptor modulators, melanocyte stimulating hormone, corticotrophin releasing factor, galanin and gamma amino butyric acid (GABA).

The compounds of the present invention are also useful as standard or reference compounds, for example as a quality standard or control, in tests or assays involving the FPR2. Such compounds may be provided in a commercial kit, for example, for use in pharmaceutical research involving FPR2 activity. For example, a compound of the present invention could be used as a reference in an assay to compare its known activity to a compound with an unknown activity. This would ensure the experimenter that the assay was being performed properly and provide a basis for comparison, especially if the test compound was a derivative of the reference compound. When developing new assays or protocols, compounds according to the present invention could be used to test their effectiveness. The compounds of the present invention may also be used in diagnostic assays involving FPR2.

The compounds of the present invention may be used in an article of manufacture. As used herein, article of manufacture is intended to include, but not be limited to, kits and packages. The article of manufacture may comprise: (a) a first container; (b) a pharmaceutical composition located within the first container, wherein the composition, comprises a first therapeutic agent, comprising a compound of the present invention or a pharmaceutically acceptable salt form thereof; and, (c) a package insert stating that the pharmaceutical composition can be used for the treatment of dyslipidemias and the sequelae thereof. The package insert may state that the pharmaceutical composition can be used in combination (as defined previously) with a second therapeutic agent for the treatment of dyslipidemias and the sequelae thereof. The article of manufacture can further comprise: (d) a second container, wherein components (a) and (b) are located within the second container and component (c) is located within or outside of the second container. Located within the first and second containers means that the respective container holds the item within its boundaries. The first container is a receptacle used to hold a pharmaceutical composition. This container can be for manufacturing, storing, shipping, and/or individual/bulk selling. First container is intended to cover a bottle, jar, vial, flask, syringe, tube (*e.g.,* for a cream preparation), or any other container used to manufacture, hold, store, or distribute a pharmaceutical product. The second container is one used to hold the first container and, optionally, the package insert. Examples of the second container include, but are not limited to, boxes (*e.g.*, cardboard or plastic), crates, cartons, bags (*e.g.,* paper or plastic bags), pouches, and sacks. The package insert can be physically attached to the outside of the first container via tape, glue, staple, or another method of attachment, or it can rest inside the second container without any physical means of attachment to the first container. Alternatively, the package insert is located on the outside of the second container. When located on the outside of the second container, it is preferable that the package insert is physically attached via tape, glue, staple, or another method of attachment. Alternatively, it can be adjacent to or touching the outside of the second container without being physically attached. The package insert is a label, tag, marker, etc. that recites information relating to the pharmaceutical composition located within the first container. The information recited will usually be determined by the regulatory agency governing the area in which the article of manufacture is to be sold (*e.g.*, the United States Food and Drug Administration). Preferably, the package insert specifically recites the indications for which the pharmaceutical composition has been approved. The package insert may be made of any material on which a person can read information contained therein or thereon. Preferably, the package insert is a printable material (*e.g.*, paper, plastic, cardboard, foil, adhesive-backed paper or plastic, etc.) on which the desired information has been formed (*e.g.*, printed or applied).

### CHEMISTRY METHODS

Abbreviations as used herein, are defined as follows: "1x" for once, "2x" for twice, "3x" for thrice, " °C" for degrees Celsius, "aq" for aqueous, "Col" for column, "eq" for equivalent or equivalents, "g" for gram or grams, "mg" for milligram or milligrams, "L" for liter or liters, "mL" for milliliter or milliliters, "µL" for microliter or microliters, "N" for normal, "M" for molar, "nM" for nanomolar, "mol" for mole or moles, "mmol" for millimole or millimoles, "min" for minute or minutes, "h" for hour or hours, "rt" for room temperature, "RT" for retention time, "ON" for overnight, "atm" for atmosphere, "psi" for pounds per square inch, "conc." for concentrate, "aq" for "aqueous", "sat" or "sat'd " for saturated, "MW" for molecular weight, "mw" or "µwave" for microwave, "mp" for melting point, "Wt" for weight, "MS" or "Mass Spec" for mass spectrometry, "ESI" for electrospray ionization mass spectroscopy, "HR" for high resolution, "HRMS" for high resolution mass spectrometry, "LCMS" for liquid chromatography mass spectrometry, "HPLC" for high pressure liquid chromatography, "RP HPLC" for reverse phase HPLC, "TLC" or "tlc" for thin layer chromatography, "NMR" for nuclear magnetic resonance spectroscopy, "nOe" for nuclear Overhauser effect spectroscopy, "¹H" for proton, "δ" for delta, "s" for singlet, "d" for doublet, "t" for triplet, "q" for quartet, "m" for multiplet, "br" for broad, "Hz" for hertz, and "α", "β", "R", "S", "E", and "Z" are stereochemical designations familiar to one skilled in the art.

| | |
|---|---|
| Ac | acetic |
| AcOH | acetic acid |
| Acn (or MeCN) | acetonitrile |
| BINAP | 2,2'-bis(diphenylphosphino)-1,1'-binaphthyl |
| BISPIN | ais(pinacolato)diboron |
| Bn | benzyl |
| Boc | *tert*-butyl carbonyl |
| Boc₂O | di-*tert*-butyl dicarbonate |
| Bu | butyl |
| dba as in (Pd₂(dba)₃) | dibenzylideneacetone |
| DCM | dichloromethane |
| DEAD | diethyl azodicarboxylate |
| DIAD | diisopropyl azodicarboxylate |
| DIEA | diisopropylethylamine |
| DMAP | 4-dimethylaminopyridine |
| DME | dimethoxyethane |
| DMF | dimethylformamide |
| DMSO | dimethyl sulfoxide |
| dppf | 1,1'-bis(diphenylphosphino)ferrocene |
| Et | ethyl |
| EtOH | ethanol |
| EtOAc | ethyl acetate |
| HATU | 2-(7-Aza-1H-benzotriazole-1-yl)-1,1,3,3-tetramet hyluronium hexafluorophosphate |
| HBTU | 2-(1*H-*Benzotriazole-1-yl)-1,1,3,3-tetramethyluro nium hexafluorophosphate |
| *i*-Bu | isobutyl |
| *i*-Pr | isopropyl |
| LAH | lithium aluminum hydride |
| Me | methyl |
| MeOH | methanol |
| NBS | N-bromosuccinimide |
| NMM | *N*-methylmorpholine |
| NMP | N-Methylpyrrolidone |
| Pet | petroleum |
| Ph | phenyl |
| Pr | propyl |
| rt | room temperature |
| *t*-Bu | *tert*-butyl |
| TBDMS-Cl | *t*-butyldimethylchlorosilane |
| TEA | triethylamine |
| TFA | trifluoroacetic acid |
| THF | tetrahydrofuran |
| Ts | tosyl |
| Xantphos | 4,5-Bis(diphenylphosphino)-9,9-dimethylxanthene |
| X-Phos | 2-Dicyclohexylphosphino-2',4',6'-triisopropylbiphenyl |

The disclosed compounds can be made by various methods known in the art including those of the following schemes and in the specific embodiments section. The structure numbering and variable numbering shown in the synthetic schemes are distinct from and should not be confused with the structure or variable numbering in the claims or the rest of the specification. The variables in the schemes are meant only to illustrate how to make some of the compounds of this invention.

The disclosure is not limited to the foregoing illustrative examples and the examples should be considered in all respects as illustrative and not restrictive, and all changes which come within the meaning and range of equivalency of the claims are therefore intended to be embraced.

A consideration in the planning of any synthetic route in this field is the choice of the protecting group used for protection of the reactive functional groups present in the compounds described in this invention. An authoritative account describing the many alternatives to the trained practitioner is Greene, T.W. et al., Protecting Groups in Organic Synthesis, 4th Edition, Wiley (2007)).

Compounds having the general Formula (I) can be prepared by the following one or more of the synthetic Schemes.

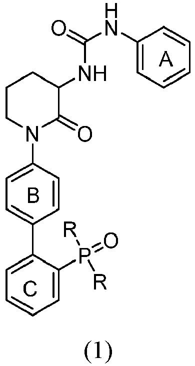

1-Arylpiperidinone compounds of this invention wherein rings A, B and C are substituted phenyl can be prepared by the general route shown in Scheme 1, starting from a suitably protected 3-aminopiperidin-2-one **1a**, where PG is a protecting group such as Boc or Cbz. Copper or palladium-catalyzed coupling of **1a** to a substituted bromobenzene **1b** in a suitable solvent such as butanol or dioxane, in the presence of a base such as potassium carbonate or cesium carbonate and a suitable ligand such as N,N'-dimethylethylenediamine or xantphos, can afford 1-phenylpiperidinones **1c.** Additional methods for this transformation include other variations of Ullmann, Goldberg, Buchwald copper-catalyzed amidation or Buchwald Pd-catalyzed amidation depending on the nature of B, using methods known to one skilled in the art for these types of couplings (see for example Yin & Buchwald Organic Lett. 2000, 2, 1101; Klapers et al. JACS, 2001, 123, 7727; Klapars et al. JACS, 2002, 124, 7421; Yin & Buchwald JACS. 2002, 124, 6043; Kiyomor, Madoux & Buchwald, Tet. Lett., 1999, 40, 2657). Subsequent palladium or iridium-catalyzed C-H borylation of 1c according to the method of Ishiyama et al. (Angew. Chem. Int. Ed. 2002, 41, 3056) to boronate **1d**, followed by condensation of the resulting pinacolatoboron species with aryl or heteroaryl halides **1e** using palladium or copper catalyzed processes provides biaryl compounds **1f**. Removal of the Boc or Cbz protecting group from **1f**, followed by condensation of the resulting free amine with a suitably substituted phenyl isocyanate, **1g** can provide ureas **1h.** Suitable isocyanates are either commercially available or can be readily obtained from the corresponding aniline by methods known to one skilled in the art. It will also be recognized by one skilled in the art that additional compounds of this invention wherein rings A, B or C are heteroaryl rings, such as pyridine, pyrimidine, thiazole, etc., can also be prepared using the methods outlined in Scheme 1 by substituting the appropriate heteroaryl iodide or bromine for **1b**, and heteroaryl isocyanate for **1g.** The racemic ureas **1h** were further subjected to enantiomeric separation using either chiral HPLC or SFC to provide **1i** and **1j** as single enantiomers.

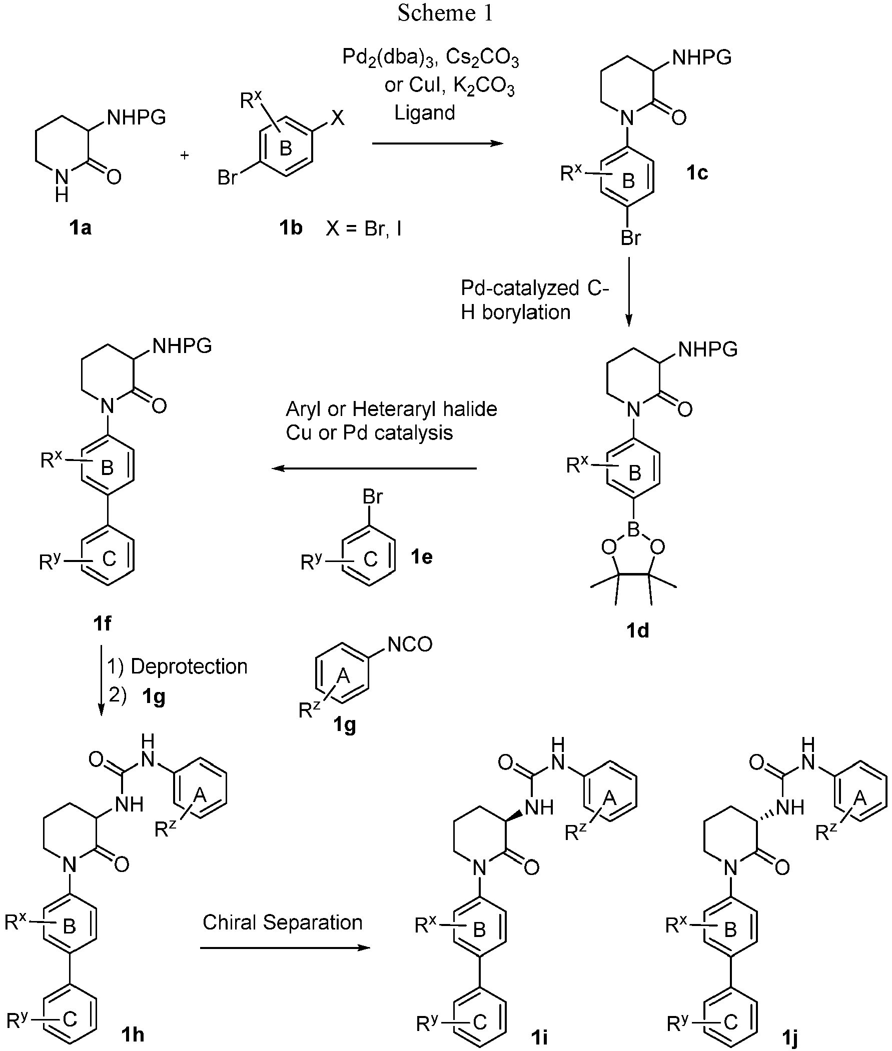

Alternatively as described in Scheme 2, compounds of this invention can be prepared from intermediate **1c** by performing palladium-catalyzed coupling to a suitably substituted phenyl boronic acid **2a,** or analogous boronate or trifluoroborate reagent, can provide the biaryl compounds **1f**. Subsequent transformations to obtain urea compounds **1h** are similar to Scheme 1.

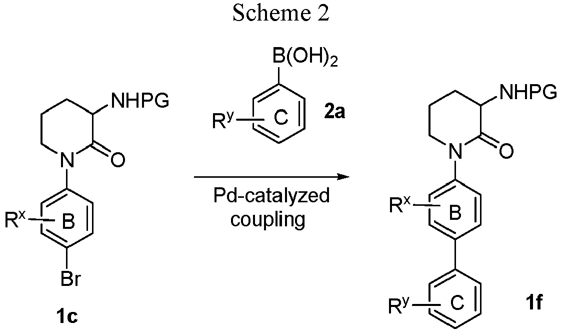

Additionally, compounds of this invention can be prepared from commercially available substituted anilines **3a** or can be readily obtained by methods known to one skilled in the art by conversion to intermediate **3c** using amide coupling with protected (*R*)-2-amino-5-(benzyloxy)-5-oxopentanoic acid. Subsequent reduction of benzyloxy ester to corresponding alcohol **3d** can be obtained using literature protocols, followed by intramolecular Mitsunobu reaction to provide compounds **1c.** Subsequent transformations to obtain urea compounds **1i** are similar to Scheme 1.

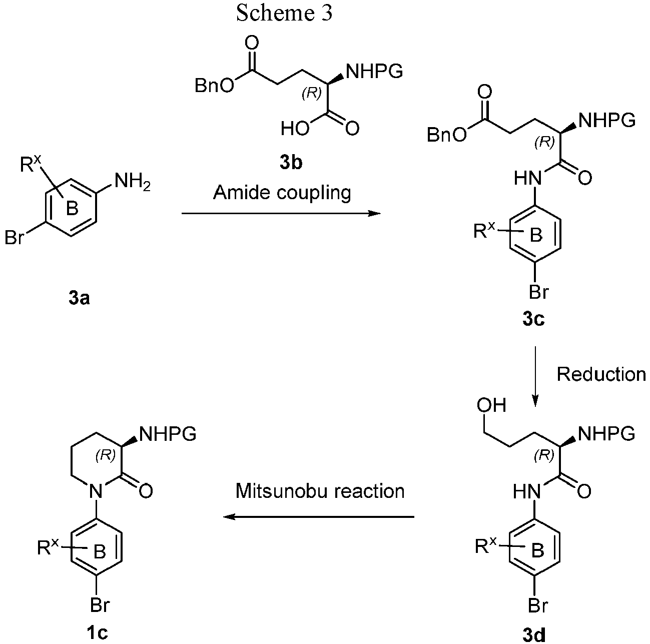

Other features of the invention will become apparent in the course of the following descriptions of exemplary embodiments that are given for illustration of the invention and are not intended to be limiting thereof.

The following methods were used in the exemplified Examples, except where noted otherwise. Purification of intermediates and final products was carried out via either normal or reverse phase chromatography. Normal phase chromatography was carried out using prepacked SiO₂ cartridges eluting with either gradients of hexanes and EtOAc or DCM and MeOH unless otherwise indicated. Reverse phase preparative HPLC was carried out using C18 columns with UV 220 nm or prep LCMS detection eluting with gradients of Solvent A (90% water, 10% MeOH, 0.1% TFA) and Solvent B (10% water, 90% MeOH, 0.1% TFA) or with gradients of Solvent A (95% water, 5% Acn, 0.1% TFA) and Solvent B (5% water, 95% Acn, 0.1% TFA) or with gradients of Solvent A (95% water, 2% Acn, 0.1% HCOOH) and Solvent B (98% Acn, 2% water, 0.1% HCOOH) or with gradients of Solvent A (95% water, 5% Acn, 10 mM NH₄OAc) and Solvent B (98% Acn, 2% water, 10 mM NH₄OAc) or with gradients of Solvent A (98% water, 2% Acn, 0.1% NH₄OH) and Solvent B (98% Acn, 2% water, 0.1% NH₄OH).
LC/MS Methods Employed in Characterization of Examples. Reverse phase analytical HPLC/MS was performed on a Waters Acquity system coupled with a Waters MICROMASS^{®} ZQ Mass Spectrometer.
Method A: Linear gradient of 0 to 100% B over 3 min, with 0.75 min hold time at 100% B;
   UV visualization at 220 nm
   Column: Waters BEH C18 2.1 x 50 mm
   Flow rate: 1.0 mL/min
   Solvent A: 0.1% TFA, 95% water, 5% Acn
   Solvent B: 0.1% TFA, 5% water, 95% Acn
Method B: Linear gradient of 0 to 100% B over 3 min, with 0.75 min hold time at 100% B;
   UV visualization at 220 nm
   Column: Waters BEH C18 2.1 x 50 mm
   Flow rate: 1.0 mL/min
   Solvent A: 10 mM ammonium acetate, 95% water, 5% Acn
   Solvent B: 10 mM ammonium acetate, 5% water, 95% Acn
   Analytical HPLC: Methods Employed in Characterization of Examples Products were analyzed by reverse phase analytical HPLC: carried out on a Shimadzu Analytical HPLC: system running Discovery VP software. RT = retention time.
Method C: Ascentis Express C18, 2.1 x 50 mm, 2.7-µm particles; Solvent A: 95% water, 5% Acn, 0.05% TFA; Solvent B: 95% Acn, 5% water, 0.1% TFA; Temperature: 50 °C; Gradient: 0-100% B over 3 minutes, then a 1-minute hold at 100% B; Flow: 1.1 mL/min. Method D: Ascentis Express C18, 2.1 x 50 mm, 2.7-µm particles; Solvent A: 95% water, 5% Acn with 10 mM ammonium acetate; Solvent B: 95% Acn, 5% water with 10 mM ammonium acetate; Temperature: 50 °C; Gradient: 0-100% B over 3 minutes, then a 1-minute hold at 100% B; Flow: 1.1 mL/min.

### SFC and chiral purity methods

Method I: Chiralpak AD-H, 250 x 4.6 mm, 5.0-µm particles; % CO₂: 60%, % Cosolvent: 40% {0.2% DEA in IPA:Acn (1:1)}, Total Flow: 4.0g/min, Back Pressure: 100 bars, Temperature : 25°C, UV: 218 nm.

Method II: Chiralpak OD-H, 250 x 4.6 mm, 5.0-µm particles; % CO₂: 60%, % Cosolvent: 40% {0.2% DEA in IPA:Acn (1:1)}, Total Flow: 4.0g/min, Back Pressure: 104 bars, Temperature : 24.9°C, UV: 287 nm.

Method III: Chiralpak OJ-H, 250 x 4.6 mm, 5.0-µm particles; % CO₂: 60%, % Cosolvent: 30% (0.3% DEA in Methanol), Total Flow: 4.0g/min, Back Pressure: 101 bars, Temperature : 23.6°C, UV: 272 nm.

Method IV: Chiralpak AS-H, 250 x 4.6 mm, 5.0-µm particles; % CO₂: 60%, % Cosolvent: 40% (0.3% DEA in Methanol), Total Flow: 4.0g/min, Back Pressure: 102 bars, Temperature : 25.4°C, UV: 272 nm.

Method V: Chiralcel OJ-H, 250 x 4.6 mm, 5.0-µm particles; % CO₂: 60%, % Cosolvent: 40% (0.2% DEA in Methanol), Total Flow: 4.0g/min, Back Pressure: 102 bars, Temperature: 24.6°C, UV: 272 nm.

Method VI: Luxcellulose-2, 250 x 4.6 mm, 5.0-µm particles; % CO₂: 60%, % Cosolvent: 35% (0.2% DEA in Methanol), Total Flow: 3.0 g/min, Back Pressure: 101 bars, Temperature: 23.6°C, UV: 260 nm.

Method VII: Chiralcel AS-H, 250 x 4.6 mm, 5.0-µm particles; % CO₂: 60%, % Cosolvent: 40% (0.2% DEA in Methanol), Total Flow: 4.0 g/min, Back Pressure: 101 bars, Temperature: 24.4°C, UV: 270 nm.

Method VIII: Chiralpak IC, 250 x 4.6 mm, 5.0-µm particles; % CO₂: 60%, % Cosolvent: 40% (0.2% DEA in Methanol), Total Flow: 4.0 g/min, Back Pressure: 101 bars, Temperature: 24.4°C, UV: 270 nm.

Method IX: column: chiralpakIF (250 X 4.6mm), 5 micron, mobile phase: -0.2% DEA in ethanol, flow: 1.0 mL/min.

Method X: column: LUX AMYLOSE 2 (250 X 4.6mm), 5 micron, mobile phase: 0.2% DEA in n-hexane:ethanol: 5:95, flow: 1.0 mL/min.

Method XI: column: CHIRALCEL OD-H (250 X 4.6mm), 5 micron, mobile phase: 0.2% DEA in n-hexane:ethanol: 70:30, flow: 1.0 mL/min.

Method XII: column: CHIRAL PAK ID 250 X 4.6mm), 5 micron, mobile phase: 0.1% DEA in methanol, flow: 1.0 mL/min.

NMR Employed in Characterization of Examples. ¹H NMR spectra were obtained with Bruker or *J*EOL^{®} Fourier transform spectrometers operating at frequencies as follows: ¹H NMR: 300 MHz (Bruker or *J*EOL^{®}) or 400 MHz (Bruker or *J*EOL^{®}) or 500 MHz (Bruker or *J*EOL^{®}). ¹³C NMR: 100 MHz (Bruker or *J*EOL^{®}). Spectra data are reported in the format: chemical shift (multiplicity, coupling constants, and number of hydrogens). Chemical shifts are specified in ppm downfield of a tetramethylsilane internal standard (δ units, tetramethylsilane = 0 ppm) and/or referenced to solvent peaks, which in ¹H NMR spectra appear at 2.49 ppm for CD₂HSOCD₃, 3.30 ppm for CD₂HOD, 1.94 for CD₃CN, and 7.24 ppm for CHCl₃, and which in ¹³C NMR spectra appear at 39.7 ppm for CD₃SOCD₃, 49.0 ppm for CD₃OD, and 77.0 ppm for CDCl₃. All ¹³C NMR spectra were proton decoupled.

### Intermediate 1: tert-Butyl (1-(4-bromo-2,3-difluorophenyl)-2-oxopiperidin-3-yl)carbamate

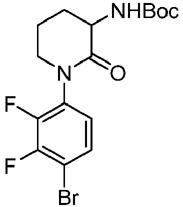

To a stirred solution of 1,4-dibromo-2,3-difluorobenzene (4.0 g, 15 mmol) in 1,4-dioxane (40 mL), were added *tert*-butyl (2-oxopiperidin-3-yl)carbamate (3.5 g, 16 mmol), and cesium carbonate (9.6 g, 29 mmol). The reaction mixture was purged with nitrogen for 5 min and charged with Xantphos (0.85 g, 1.5 mmol) and Pd₂(dba)₃ (0.67 g, 0.74 mmol). The reaction mixture was again purged with nitrogen for 3 min and heated at 110 °C for 16 h. The mixture was cooled, filtered through a Celite pad and the filtrate was concentrated under reduced pressure. The crude product was purified via column chromatography (pet. ether-EtOAc) to afford the Intermediate 1 (1.9 g, 4.7 mmol, 20% yield) as a yellowish solid. MS(ESI) m/z: 405.2 (M+H)⁺. ¹H NMR (400 MHz, CDCl₃) *δ* 7.36 (m, 1H), 6.96 (m, 1H), 5.44 (s, 1H), 4.37 - 4.22 (m, 1H), 3.71 - 3.58 (m, 2H), 2.69 - 2.55 (m, 1H), 2.16 - 2.02 (m, 2H), 1.77 (m, 1H), 1.47 (m, 9H).

### Intermediate 2: tert-Butyl (1-(2,3-difluoro-4-(4,4,5,5-tetramethyl-1,3,2-dioxaborolan-2-yl)phenyl)-2-oxopiperidin-3-yl)carbamate

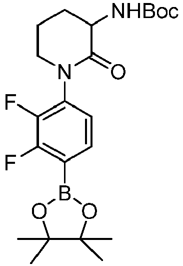

To a stirred solution of Intermediate 2 (250 mg, 0.62 mmol) in 1,4-dioxane (10 mL), were added bis(pinacolato)diboron (240 mg, 0.93 mmol) and potassium acetate (150 mg, 1.5 mmol). The resulting reaction mixture was purged with argon for 5 min and charged with 1,1'-bis(diphenylphosphino)ferrocene-palladium(II)dichloride.DCM complex (35 mg, 0.043 mmol). Then, the reaction mixture was again purged with argon for 3 min and heated to 80 °C for 16 h. The reaction mixture was filtered through a Celite pad, concentrated under reduced pressure and used for the next step without further purification. MS(ESI) m/z: 453.5 (M+H)⁺.

### Intermediate 3: tert-Butyl (1-(2'-(dimethylphosphoryl)-2,3-difluoro-[1,1'-biphenyl]-4-yl)-2-oxopiperidin-3-yl)carbamate

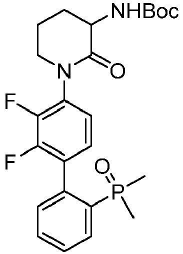

To a stirred solution of Intermediate 2 (230 mg, 0.50 mmol) in 1,4-dioxane (2 mL), were added (2-bromophenyl)dimethylphosphine oxide (120 mg, 0.50 mmol), and potassium phosphate (210 mg, 1.0 mmol). The reaction mixture was purged with nitrogen for 5 min and charged with 1,1'-bis(diphenylphosphino)ferrocene-palladium(II)dichloride.DCM complex (41 mg, 0.050 mmol). The reaction mixture was again purged with nitrogen for 3 min and heated at 80 °C for 16 h. The reaction mixture was cooled, filtered through Celite pad and the filtrate was concentrated under reduced pressure. The crude product was purified via flash chromatography (CHCl₃-MeOH) to afford the Intermediate 3 (140 mg, 0.23 mmol, 47 % yield) as a dark brown liquid. MS(ESI) m/z: 496.5 (M+NH₄)⁺. ¹H NMR (400 MHz, DMSO-d₆) δ 8.08 - 7.86 (m, 1H), 7.83 - 7.74 (m, 2H), 7.68 - 7.50 (m, 3H), 7.29 - 7.22 (m, 1H), 4.19 - 4.15 (m, 1H), 3.71 - 3.68 (m, 1H), 3.64 - 3.60 (m, 1H), 2.15 - 1.83 (m, 4H), 1.53 - 1.46 (s, 9H), 1.42 - 1.34 (m, 6H).

### Intermediate 4: 3-amino-1-(2'-(dimethylphosphoryl)-2,3-difluoro-[1,1 '-biphenyl]-4-yl)piperidin-2-one

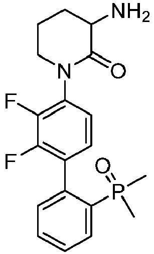

To an ice cooled solution of Intermediate 3 (130 mg, 0.27 mmol) in 1,4-dioxane (1.5 mL) under argon atmosphere at rt, was added 4 M HCl (1.0 mL, 4.0 mmol) in 1,4-dioxane and the mixture was stirred at rt for 2 h. The solvent was evaporated under reduced pressure to obtain a gummy solid. It was further triturated with pet ether (5 mL x 2) and dried to afford the Intermediate 4 (95 mg, 0.25 mmol, 92% yield) as a brown solid. MS(ESI) m/z: 379.2 (M+H)⁺.

### Intermediate 5: (2-Bromophenyl)dimethylphosphine oxide

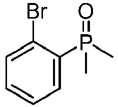

To a stirred solution of 1-bromo-2-iodobenzene (10 g, 35 mmol) in DMF (80 mL), were added dimethylphosphine oxide (3.3 g, 42 mmol), and K₃PO₄ (8.3 g, 39 mmol). The reaction mixture was purged with nitrogen for 5 min and charged with Xantphos (1.2 g, 2.1 mmol) and Pd(OAc)₂ (0.40 g, 1.8 mmol). The reaction mixture was again purged with nitrogen for 3 min and heated at 100 °C for 6 h. The reaction mixture was cooled, filtered through a Celite pad and concentrated under reduced pressure. The crude product was purified via chromatography (MeOH-DCM) to afford the Intermediate 5 (5.0 g, 21 mmol, 60% yield) as a yellowish solid. MS(ESI) m/z: 232.9 (M+H)⁺. ¹H NMR (400 MHz, DMSO-d₆) 7.98 - 7.94 (m, 1H), 7.77 - 7.74 (m, 1H), 7.76 - 7.48 (m, 2H), 1.83 (d, J = 14 Hz, 6H).

### Intermediate 6: Dimethyl(2-(4,4,5,5-tetramethyl-1,3,2-dioxaborolan-2-yl)phenyl)phosphine oxide

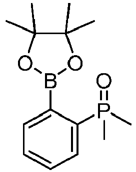

To a stirred solution of Intermediate 5 (1.0 g, 4.3 mmol), were added bis(pinacolato)diboron (1.3 g, 5.2 mmol) and sodium acetate (0.39 g, 4.7 mmol). The reaction mixture was purged with nitrogen for 5 min and charged with Pd(dba)₂ (0.025 g, 0.043 mmol) and X-phos (0.041 g, 0.086 mmol). The reaction mixture was again purged with nitrogen for 3 min and heated at 110 °C for 3 h. The reaction mixture was diluted with EtOAc (20 mL), filtered through pad of Celite pad and evaporated under reduced pressure. The crude product was used in the next step without further purification. MS(ESI) m/z: 281.0 (M+H)⁺.

### Intermediate 7: Benzyl (R)-5-((4-bromo-2,6-difluorophenyl)amino)-4-((tert-butoxycarbonyl)amino)-5-oxopentanoate

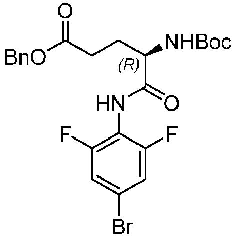

To a stirred solution of Intermediate 6 (5.0 g, 15 mmol) in Acn (40 mL) under argon atmosphere at rt, were added DIEA (7.8 mL, 45 mmol), HATU (6.8 g, 18 mmol) and 4-bromo-2,6-difluoroaniline (3.2 g, 16 mmol). The reaction mixture was stirred at 60 °C for 16 h. Then, the reaction mixture was concentrated under reduced pressure, quenched with aqueous saturated NH₄Cl (10 mL) solution and extracted with EtOAc (20 mL x 3). The combined organic layers were washed with brine (20 mL), dried over Na₂SO₄, concentrated under reduced pressure and purified via column chromatography (35% EtOAc - Pet.-ether) to afford Intermediate 7 (1.4 g, 2.7 mmol, 18% yield) as a white solid. MS(ESI) m/z: 527.1 (M+H)⁺. ¹H NMR (300MHz, CDCl₃) *δ* 7.36 - 7.35 (m, 5H), 7.16 (d, *J =* 5.4 Hz, 2H), 5.35 - 5.30 (m, 1H), 5.15 - 5.11 (m, 2H), 2.72 - 2.48 (m, 2H), 2.31 - 2.19 (m, 1H), 2.10 - 1.99 (m, 1H), 1.54 (s, 9H), 1.53 - 1.50 (m, 1H), 0.90 - 0.84 (m, 1H).

### Intermediate 8: tert-Butyl (R)-(1-((4-bromo-2,6-difluorophenyl)amino)-5-hydroxy-1-oxopentan-2-yl)carbamate

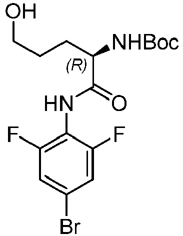

To a stirred solution of Intermediate 7 (1.4 g, 2.7 mmol) in THF (12 mL) under argon at 0 °C, were added MeOH (0.32 mL, 8.0 mmol) and LiBH₄ (4.0 mL, 8.0 mmol). The resulting reaction mixture was gradually warm up to rt and stirred for an additional 30 min. The reaction mixture was quenched with aqueous NH₄Cl (15 mL) in a drop wise manner and extracted with EtOAc (20 mL x 3). The combined organic layers were washed with water (15 mL) and brine (20 mL), dried over Na₂SO₄, and concentrated under reduced pressure. The crude product was purified via column chromatography (70% EtOAc-Pet. ether) to afford Intermediate 8 (860 mg, 2.0 mmol, 77% yield) as a white solid. MS(ESI) m/z: 423.1 (M+H)⁺. ¹H NMR (400 MHz, CDCl₃) *δ* 8.28 (br s, 1H), 7.17 - 7.12 (m, 2H), 5.36 (br s, 1H), 4.52 - 4.48 (m, 1H), 3.79 (q, *J* = 5.5 Hz, 2H), 2.10 - 1.97 (m, 1H), 1.94 - 1.68 (m, 3H), 1.52 - 1.44 (m, 9H).

### Intermediate 9: tert-Butyl (R)-(1-(4-bromo-2,6-difluorophenyl)-2-oxopiperidin-3-yl)carbamate

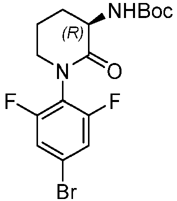

To an ice cooled solution of di-tert-butyl azodicarboxylate (1.0 g, 4.4 mmol) in THF (5 mL) under argon atmosphere at rt, was added *tri-n*-butylphosphine (1.1 mL, 4.4 mmol). After 5 min, an ice cold solution of Intermediate 8 (1.3 g, 3.0 mmol) in THF (5 mL) was cannulated into the flask and the reaction mixture was warmed to rt over 2 h. The reaction mixture was quenched with aq. saturated NaHCO₃ solution (20 mL) and extracted with EtOAc (20 mL x 3). The combined organic layers were washed with aq. NH₄Cl solution (15 mL x 2) and brine (20 mL), dried over Na₂SO₄, and concentrated under reduced pressure. The crude product was purified via column chromatography (20% pet. ether-EtOAc) to afford Intermediate 9 (800 mg, 2.0 mmol, 67% yield) as white solid. MS(ESI) m/z: 405.1 (M+H)⁺. ¹H NMR (400 MHz, CDCl₃) *δ* 7.20 - 7.18 (m, 1H), 7.16 - 7.12 (m, 1H), 5.44 (br s, 1H), 4.36 - 4.24 (m, 1H), 3.66 - 3.51 (m, 2H), 2.66 - 2.56 (m, 1H), 2.12 - 2.05 (m, 2H), 1.81 - 1.70 (m, 1H), 1.57 - 1.45 (s, 9H).

### Example 1: (R)-1-(4-Chloro-2-fluorophenyl)-3-(1-(2'-(dimethylphosphoryl)-2,3-difluoro-[1,1'-biphenyl]-4-yl)-2-oxopiperidin-3-yl)urea

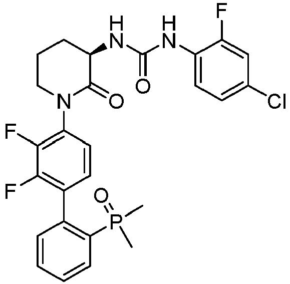

To an ice cooled suspension of Intermediate 4 (100 mg, 0.26 mmol) in DMF (2 mL) under argon atmosphere at rt, were added DIEA (0.14 mL, 0.79 mmol) and phenyl (4-chloro-2-fluorophenyl)carbamate (70 mg, 0.26 mmol). The resulting solution was heated at 45 °C for 16 h. The reaction mixture was concentrated under reduced pressure and the residue was purified by reverse phase HPLC followed by chiral HPLC to afford Example 1 (22 mg, 0.039 mmol, 15% yield) as white solid. MS(ESI) m/z: 550.2 (M+H)⁺. ¹H NMR (400 MHz, DMSO-d₆) *δ* 8.67 (d, *J* = 2.2 Hz, 1H), 8.17 (t, *J* = 8.9 Hz, 1H), 8.01 - 7.88 (m, 1H), 7.74 - 7.57 (m, 2H), 7.50 - 7.33 (m, 2H), 7.33 - 7.21 (m, 2H), 7.21 - 7.05 (m, 2H), 4.48 - 4.37 (m, 1H), 3.85 - 3.62 (m, 2H), 2.31 (dd, *J* = 12.3, 6.5 Hz, 1H), 2.17 - 1.93 (m, 2H), 1.86 (d, *J* = 6.5 Hz, 1H), 1.49 (d, *J* = 13.5 Hz, 6H). RT = 1.539 min, 99.2% (Method D).

The following Examples in Table 2 were made by using the same procedure as shown in Example 1.

| **Example No** | **Structure** | **IUPAC Name** | **LCMS (M+H)⁺** | **HPLC Method, RT (min.) & Purity** |
|---|---|---|---|---|
| **2** | 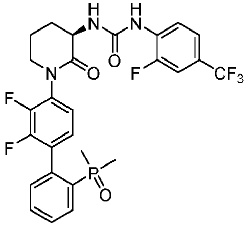 | 3-[(3*R*)-1-[2'-(Dimethylphosphoryl)-2,3-difluoro-[1,1'-biphenyl]-4-yl]-2-oxopiperidin-3-yl]-1-[2-fluoro-4-(trifluoromethyl)phenyl]ur ea | 584.2 | Method D, RT = 1.946 min, 99.8% |
| **3** | 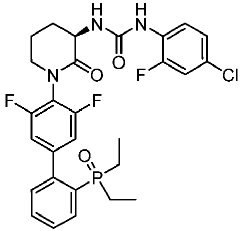 | 1-(4-Chloro-2-fluorophenyl)-3-[(3*R*)-1 - [2'-(diethylphosphoryl)-3,5-difluoro-[1,1'-biphenyl]-4-yl]-2-oxopiperidin-3-yl]urea | 578.2 | Method D, RT = 1.957 min, 100% |
| **4** | 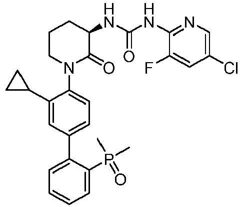 | 1-(5-Chloro-3-fluoropyridin-2-yl)-3 - [(3*R*)-1-[3 -cyclopropyl-2'-(dimethylphosphoryl)-[1,1'-biphenyl]-4-yl]-2-oxopiperidin-3-yl]urea | 555.2 | Method D, RT = 1.776 min, 97.8% |
| **5** | 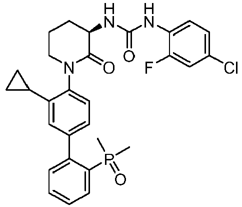 | 1-(4-Chloro-2-fluorophenyl)-3-[(3*R*)-1- [3-cyclopropyl-2'-(dimethylphosphoryl)-[1,1'-biphenyl]-4-yl]-2-oxopiperidin-3-yl]urea | 571.3 (M+NH₄ )⁺ | Method D, RT = 1.957 min, 100% |
| **6** | 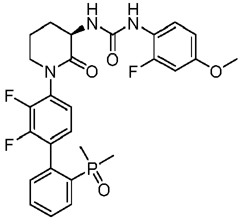 | 3-[(3*R*)-1-[2'-(Dimethylphosphoryl)-2,3-difluoro-[1,1'-biphenyl]-4-yl]-2-oxopiperidin-3-yl]-1-(2-fluoro-4-methoxyphenyl)urea | 546.2 | Method D, RT = 1.445 min, 100% |
| **7** | 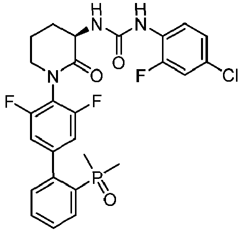 | 1-(4-Chloro-2-fluorophenyl)-3-[(3*R*)-1- [2'-(dimethylphosphoryl)-3,5-difluoro-[1,1'-biphenyl]-4-yl]-2-oxopiperidin-3-yl]urea | 550.2 | Method D, RT = 1.957 min, 100% |
| **8** | 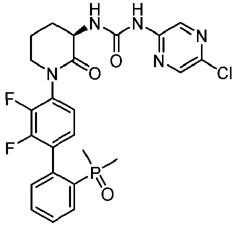 | 1-(5-Chloropyrazin-2-yl)-3-[(3*R*)-1-[2'-(dimethylphosphoryl)-2,3-difluoro-[1,1'-biphenyl]-4-yl]-2-oxopiperidin-3-yl]urea | 534.2 | Method C, RT = 1.569 min, 100% |
| **9** | 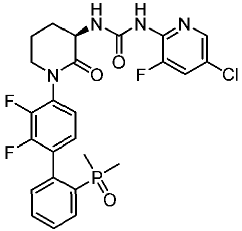 | 1-(5-Chloro-3-fluoropyridin-2-yl)-3- [(3*R*)-1-[2'-(dimethylphosphoryl)-2,3-difluoro-[1,1'-biphenyl]-4-yl]-2-oxopiperidin-3-yl]urea | 551.2 | Method C, RT = 1.691 min, 99.6% |
| **10** | 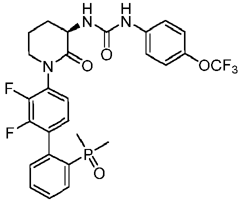 | 3-[(3*R*)-1-[2'-(Dimethylphosphoryl)-2,3-difluoro-[1,1'-biphenyl]-4-yl]-2-oxopiperidin-3-yl]-1-[4-(trifluoromethoxy)phenyl] urea | 582.2 | Method D, RT = 1.909 min, 99.7% |
| **11** | 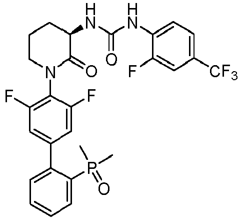 | 3-[(3*R*)-1-[2'-(Dimethylphosphoryl)-3,5-difluoro-[1,1'-biphenyl]-4-yl]-2-oxopiperidin-3-yl]-1-[2-fluoro-4-(trifluoromethyl)phenyl]ur ea | 584.3 | Method C, RT = 1.797 min, 99.7% |
| **12** | 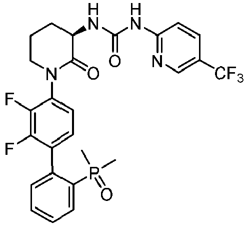 | 3-[(3*R*)-1-[2'-(Dimethylphosphoryl)-2,3-difluoro-[1,1'-biphenyl]-4-yl]-2-oxopiperidin-3-yl]-1-[5-(trifluoromethyl)pyridin-2-yl]urea | 567.2 | Method C, RT = 1.797 min, 100% |
| **13** | 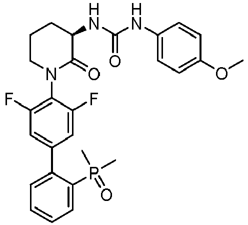 | 3-[(3*R*)-1-[2'-(Dimethylphosphoryl)-3,5-difluoro-[1,1'-biphenyl]-4-yl]-2-oxopiperidin-3-yl]-1-(4-methoxyphenyl)urea | 528.3 | Method C, RT = 1.378 min, 100% |
| **14** | 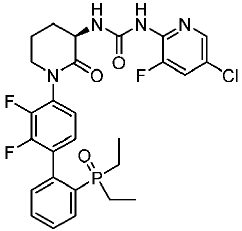 | 1-(5-Chloro-3-fluoropyridin-2-yl)-3-[(3*R*)-1-[2'-(diethylphosphoryl)-2,3-difluoro-[1,1'-biphenyl]-4-yl]-2-oxopiperidin-3-yl]urea | 579.3 | Method C, RT = 1.378 min, 100% |
| **15** | 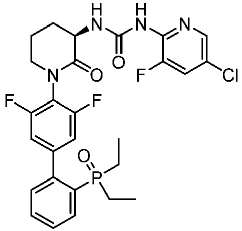 | 1-(5-Chloro-3-fluoropyridin-2-yl)-3- [(3*R*)-1-[2'-(diethylphosphoryl)-3,5-difluoro-[1,1'-biphenyl]-4-yl]-2-oxopiperidin-3-yl]urea | 579.2 | Method D, RT = 1.378 min, 100% |
| **16** | 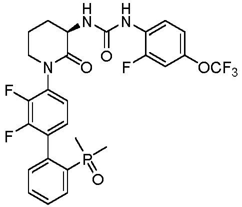 | 3-[(3*R*)-1-[2'-(Dimethylphosphoryl)-2,3-difluoro-[1,1'-biphenyl]-4-yl]-2-oxopiperidin-3-yl]-1-[2-fluoro-4-(trifluoromethoxy)phenyl] urea | 617.3 (M+NH₄ )⁺ | Method D, RT = 1.790 min, 98.6% |
| **17** | 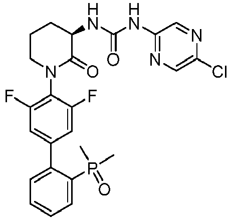 | 1-(5-Chloropyrazin-2-yl)-3-[(3*R*)-1-[2'-(dimethylphosphoryl)-3,5 - difluoro-[1,1'-biphenyl]-4-yl]-2-oxopiperidin-3-yl]urea | 534.2 | Method C, RT = 1.401 min, 100% |
| **18** | 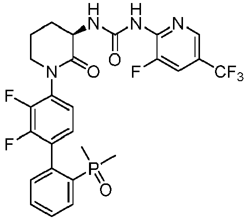 | 3-[(3*R*)-1-[2'-(Dimethylphosphoryl)-2,3-difluoro-[1,1'-biphenyl]-4-yl]-2-oxopiperidin-3-yl]-1-[3-fluoro-5-(trifluoromethyl)pyridin-2-yl]urea | 585.3 | Method C, RT = 1.804 min, 99.8% |
| **19** | 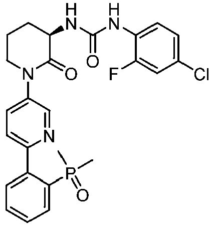 | 1-(4-Chloro-2-fluorophenyl)-3-[(3*R*)-1- {6-[2-(dimethylphosphoryl)phen yl]pyridin-3-yl}-2-oxopiperidin-3-yl]urea | 515.2 | Method D, RT = 1.565 min, 100% |
| **20** | 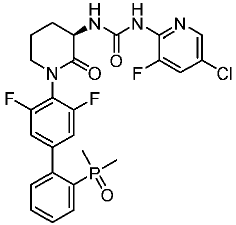 | 1-(5-Chloro-3-fluoropyridin-2-yl)-3-[(3*R*)-1-[2'-(dimethylphosphoryl)-3,5-difluoro-[1,1'-biphenyl]-4-yl]-2-oxopiperidin-3-yl]urea | 551.2 | Method C, RT = 1.521 min, 95.3% |
| **21** | 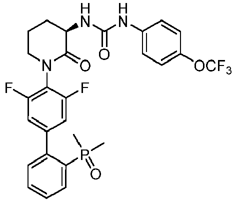 | 3-[(3*R*)-1-[2'-(Dimethylphosphoryl)-3,5-difluoro-[1,1'-biphenyl]-4-yl]-2-oxopiperidin-3-yl]-1-[4-(trifluoromethoxy)phenyl] urea | 567.1 | Method C, RT = 1.622 min, 100% |

### NMR data for Examples in Table 2:

Example 2: ¹H NMR (400 MHz, DMSO-d6) δ 8.95 (br. s., 1H), 8.42 (t, J = 8.3 Hz, 1H), 7.95 (m, 1H), 7.73 - 7.56 (m, 3H), 7.50 (d, J = 8.8 Hz, 1H), 7.39 (d, J = 3.7 Hz, 1H), 7.34 - 7.15 (m, 3H), 4.52 - 4.39 (m, 1H), 3.76 - 3.68 (m, 2H), 2.39 - 2.28 (m, 1H), 2.22 - 1.96 (m, 2H), 1.91 - 1.85 (m,1H), 1.49 (d, J = 13.5 Hz, 6H).

Example 3: ¹H NMR (400 MHz, DMSO-d6) δ 8.64 (s, 1H), 8.17 (t, J = 8.9 Hz, 1H), 7.91 (m, 1H), 7.73 - 7.44 (m, 2H), 7.44 - 7.33 (m, 2H), 7.29 (d, J = 9.0 Hz, 2H), 7.23 - 7.09 (m, 2H), 4.50 - 4.38 (m, 1H), 3.72 - 3.59 (m, 2H), 2.35 - 2.29 (m, 1H), 2.1 - 2.02 (m, 2H), 1.84 (d, J = 5.6 Hz, 1H), 1.75 - 1.51 (m, 4H), 1.07 - 0.79 (m, 6H).

Example 4: ¹H NMR (400 MHz, DMSO-d6) δ 9.38 (br. s., 1H), 8.83 (s, 1H), 8.21 - 8.09 (m, 1H), 8.02 (m, 1H), 7.94 (m, 1H), 7.61 (t, J = 7.5 Hz, 1H), 7.54 (t, J = 7.5 Hz, 1H), 7.35 (m, 1H), 7.32 - 7.14 (m, 2H), 7.10 (d, J = 7.1 Hz, 1H), 4.53 - 4.39 (m, 1H), 3.75 - 3.56 (m, 2H), 2.42 - 2.38 (m, 1H), 2.15 - 1.82 (m, 4H), 1.44 - 1.14 (m, 6H), 1.04 - 0.85 (m, 3H), 0.64 - 0.60 (m, 1H).

Example 5: ¹H NMR (400 MHz, DMSO-d6) δ 8.69 (s, 1H), 8.17 (m, 1H), 8.01 - 7.87 (m, 1H), 7.64 - 7.44 (m, 2H), 7.44 - 7.31 (m, 2H), 7.28 - 7.25 (m, 2H), 7.23 - 6.95 (m, 3H), 4.38 - 4.34 (m, 1H), 3.66 - 3.55 (m, 2H), 2.35 - 2.30 (m, 1H), 2.09 - 2.03 (m, 2H), 1.92 - 1.86 (m, 2H), 1.33 - 1.27 (m, 6H), 0.87 - 0.84 (m, 3H), 0.64 - 0.61 (m, 1H).

Example 6: ¹H NMR (400 MHz, DMSO-d6) δ 8.30 (d, J = 1.5 Hz, 1H), 7.95 (m, 1H), 7.86 (m, 1H), 7.70 - 7.55 (m, 2H), 7.38 (m, 1H), 7.33 - 7.19 (m, 2H), 6.90 (d, J = 6.8 Hz, 1H), 6.86 (m, 1H), 6.71 (m, 1H), 4.45 - 4.33 (m, 1H), 3.82 - 3.59 (m, 5H), 2.32 - 2.24 (m, 1H), 2.14 - 1.97 (m, 2H), 1.88 - 1.73 (m, 1H), 1.59 - 1.34 (m, 6H).

Example 7: ¹H NMR (400 MHz, DMSO-d6) δ 8.63 (d, J = 2.4 Hz, 1H), 8.17 (m, 1H), 7.92 (m, 1H), 7.69 - 7.52 (m, 2H), 7.49 - 7.24 (m, 4H), 7.22 - 7.05 (m, 2H), 4.50 - 4.40 (m, 1H), 3.77 - 3.58 (m, 2H), 2.32 (dd, J = 12.0, 5.9 Hz, 1H), 2.17 - 1.94 (m, 2H), 1.90 - 1.77 (m, 1H), 1.46 (d, J = 13.5 Hz, 6H).

Example 8: ¹H NMR (400 MHz, DMSO-d6) δ 8.88 (s, 2H), 8.40 (d, J = 1.2 Hz, 1H), 7.95 (m, 1H), 7.73 - 7.57 (m, 2H), 7.53 (s, 1H), 7.38 (s, 1H), 7.34 - 7.17 (m, 2H), 4.47 (m, 1H), 3.79 - 3.75 (m, 1H), 3.74 - 3.68 (m, 1H), 2.37 - 2.33 (m, 1H), 2.18 - 1.98 (m, 2H), 1.98 - 1.80 (m, 1H), 1.53 - 1.46 (m, 6H).

Example 9: ¹H NMR (400 MHz, DMSO-d6) δ 9.41 (s, 1H), 8.83 (s, 1H), 8.17 (d, J = 2.0 Hz, 1H), 8.03 (m, 1H), 7.98 - 7.90 (m, 1H), 7.72 - 7.54 (m, 2H), 7.37 (m, 1H), 7.33 - 7.18 (m, 2H), 4.56 - 4.47 (m, 1H), 3.79- 3.73 (m, 1H), 3.72 - 3.66 (m, 1H), 2.38 (m, 1H), 2.16 - 1.98 (m, 2H), 1.96 - 1.87 (m, 1H), 1.49 (d, J = 13.5 Hz, 6H).

Example 10: ¹H NMR (400 MHz, DMSO-d6) δ 8.96 (s, 1H), 8.01 - 7.89 (m, 1H), 7.73 - 7.56 (m, 2H), 7.56 - 7.41 (m, 2H), 7.41 - 7.34 (m, 1H), 7.33 - 7.14 (m, 4H), 6.62 (d, J = 7.1 Hz, 1H), 4.45 - 4.35 (m, 1H), 3.80 - 3.60 (m, 2H), 2.31 (m, 1H), 2.16 - 1.94 (m, 2H), 1.88 (m, 1H), 1.48 (d, J = 13.5 Hz, 6H).

Example 11: ¹H NMR (400 MHz, DMSO-d6) δ 8.92 (d, J = 2.9 Hz, 1H), 8.42 (m, 1H), 7.93 (m, 1H), 7.73 - 7.55 (m, 3H), 7.51 (d, J = 8.6 Hz, 1H), 7.46 - 7.35 (m, 3H), 7.33 (d, J = 7.3 Hz, 1H), 4.52 - 4.43 (m, 1H), 3.75 - 3.59 (m, 2H), 2.37 - 2.32 (m, 1H), 2.21 - 1.94 (m, 2H), 1.92 - 1.80 (m, 1H), 1.46 (d, J = 13.5 Hz, 6H).

Example 12: ¹H NMR (400 MHz, DMSO-d6) δ 9.81 (s, 1H), 8.63 - 8.52 (m, 1H), 8.13 (s, 1H), 8.07 (dd, J = 9.0, 2.4 Hz, 1H), 7.99 - 7.90 (m, 1H), 7.74 (d, J = 8.8 Hz, 1H), 7.70 - 7.56 (m, 2H), 7.43 - 7.34 (m, 1H), 7.34 - 7.22 (m, 2H), 4.54 - 4.45 (m, 1H), 3.83 - 3.74 (m, 1H), 3.74 - 3.65 (m, 1H), 2.41 - 2.30 (m, 1H), 2.17 - 1.97 (m, 2H), 1.95 - 1.79 (m, 1H), 1.48 (d, J = 13.5 Hz, 6H).

Example 13: ¹H NMR (400 MHz, DMSO-d6) δ 8.48 (s, 1H), 7.92 (m, 1H), 7.72 - 7.53 (m, 2H), 7.46 - 7.36 (m, 3H), 7.35 - 7.22 (m, 2H), 6.91 - 6.75 (m, 2H), 6.47 (d, J = 6.8 Hz, 1H), 4.42 (m, 1H), 3.70 (s, 3H), 3.68 - 3.58 (m, 2H), 2.35 - 2.27 (m, 1H), 2.18 - 1.93 (m, 2H), 1.89 - 1.78 (m, 1H), 1.46 (d, J = 13.5 Hz, 6H).

Example 14: ¹H NMR (400 MHz, DMSO-d6) δ 8.93 - 8.82 (m, 1H), 8.16 (d, J = 2.2 Hz, 1H), 8.02 (m, 1H), 7.95 - 7.86 (m, 1H), 7.69 - 7.58 (m, 3H), 7.34 (m, 1H), 7.28 (m, 1H), 7.21 - 7.14 (m, 1H), 4.55 - 4.48 (m, 1H), 3.81 - 3.74 (m, 1H), 3.72 - 3.65 (m, 1H), 2.42 - 2.34 (m, 1H), 2.18 - 1.93 (m, 2H),1.96 - 1.84 (m, 1H), 1.81 - 1.66 (m, 3H), 1.65 - 1.52 (m, 1H), 1.11 - 0.68 (m, 6H).

Example 15: ¹H NMR (400 MHz, DMSO-d6) δ 9.42 (s, 1H), 8.81 (d, J = 5.9 Hz, 1H), 8.18 (s, 1H), 8.10 - 7.98 (m, 1H), 7.90 (m, 1H), 7.74 - 7.50 (m, 2H), 7.35 (m, 1H), 7.29 (m, 2H), 4.61 - 4.48 (m, 1H), 3.68 (m, 2H), 2.43 - 2.37 (m, 1H), 2.18 - 1.99 (m, 2H), 1.97 - 1.84 (m, 1H), 1.78 - 1.54 (m, 4H), 1.03 - 0.77 (m, 6H).

Example 16: ¹H NMR (400 MHz, DMSO-d6) δ 8.74 (d, J = 2.4 Hz, 1H), 8.24 (m, 1H), 7.95 (m, 7.6, 1.3 Hz, 1H), 7.70 - 7.57 (m, 2H), 7.45 - 7.22 (m, 4H), 7.20 - 7.09 (m, 2H), 4.47 - 4.37 (m, 1H), 3.77 - 3.61 (m, 2H), 2.35 - 2.30 (m, 1H), 2.12 - 1.99 (m, 2H), 1.91 - 1.80 (m, 1H), 1.58 - 1.41 (m, 6H).

Example 17: ¹H NMR (400 MHz, DMSO-d6) δ 9.68 (s, 1H), 8.88 (d, J = 1.5 Hz, 1H), 8.40 (d, J = 1.2 Hz, 1H), 7.92 (m, 1H), 7.72 - 7.54 (m, 2H), 7.50 (d, J = 6.8 Hz, 1H), 7.45 - 7.26 (m, 3H), 4.55 - 4.45 (m, 1H), 3.77 - 3.55 (m, 2H), 2.42 - 2.34 (m, 1H), 2.21 - 1.94 (m, 2H), 1.93 - 1.80 (m, 1H), 1.46 (d, J = 13.5 Hz, 6H).

Example 18: ¹H NMR (400 MHz, DMSO-d6) δ 9.52 (s, 1H), 9.26 (s, 1H), 8.47 (s, 1H), 8.20 (m, 1H), 7.95 (m, 1H), 7.75 - 7.53 (m, 2H), 7.37 (m, 1H), 7.33 - 7.19 (m, 2H), 4.60 - 4.52 (m, 1H), 3.80 - 3.76 (m, 1H), 3.75 - 3.66 (m, 1H), 2.41 (m, 1H), 2.07 (d, J = 5.1 Hz, 2H), 1.99 - 1.88 (m, 1H), 1.49 (d, J = 13.5 Hz, 6H).

Example 19: ¹H NMR (400 MHz, DMSO-d6) δ 8.72 (br. s., 1H), 8.65 (s, 1H), 8.16 (m, 1H), 8.08 (m, 1H), 7.90 (m, 1H), 7.75 - 7.55 (m, 3H), 7.55 - 7.46 (m, 1H), 7.40 (d, J = 12.5 Hz, 1H), 7.14 (d, J = 7.6 Hz, 1H), 7.18 (d, J = 9.0 Hz, 1H), 4.40 (m, 1H), 3.85 (m, 1H), 3.79 - 3.70 (m, 1H), 2.28 (d, J = 5.9 Hz, 1H), 2.16 - 1.91 (m, 2H), 1.86 (d, J = 6.4 Hz, 1H), 1.46 (d, J = 13.5 Hz, 6H).

Example 20: ¹H NMR (400 MHz, DMSO-d6) δ 9.40- 9.15 (br. s., 1H), 8.80 (br. s., 1H), 8.18 (d, J = 2.0 Hz, 1H), 8.02 (m, 1H), 7.96 - 7.84 (m, 1H), 7.71 - 7.51 (m, 2H), 7.48 - 7.27 (m, 3H), 4.60 - 4.48 (m, 1H), 3.76 - 3.57 (m, 2H), 2.39 (m, 1H), 2.17 - 2.00 (m, 2H), 2.00 - 1.81 (m, 1H), 1.46 (d, J = 13.5 Hz, 6H).

Example 21: ¹H NMR (400 MHz, DMSO-d6) δ 9.76 (s, 1H), 8.58 (s, 1H), 8.17 - 8.04 (m, 2H), 7.96 - 7.89 (m, 1H), 7.76 (d, J=8.8 Hz, 1H), 7.65 (m, 1H), 7.59 (m, 1H), 7.47 - 7.31 (m, 3H), 4.59 - 4.49 (m, 1H), 3.68 (m, 2H), 2.37 (m, 1H), 2.20 - 1.96 (m, 2H), 1.94 - 1.81 (m, 1H), 1.46 (d, J = 13.5 Hz, 6H).

## Claims

1. A compound of Formula (I): 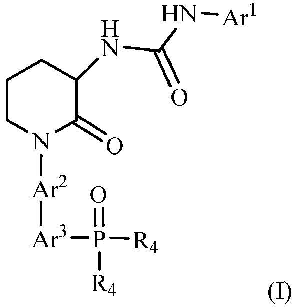 or a pharmaceutically acceptable salt thereof, wherein:
Ar¹ is phenyl substituted with 1-2 R^{1a} and 1-2 R^{1b} or 6-membered heteroaryl with 1-3 nitrogen atoms and substituted with 1-2 R^{1a} and 1-2 R^{1b};
Ar² is phenyl substituted with 1-4 R² or 6-membered heteroaryl with 1-3 nitrogen atoms and substituted with 1-4 R²;
Ar³ is phenyl substituted with 0-4 R³ or pyridinyl substituted with 0-4 R³;
R^{1a} is hydrogen or halo;
R^{1b} is halo, haloalkyl, alkoxy, or haloalkoxy;
R² is hydrogen, cyano, halo, alkyl, hydroxyalkyl, haloalkyl, cycloalkyl, alkoxy, or haloalkoxy;
R³ is cyano, halo, alkyl, alkoxy, hydroxyalkyl, alkoxyalkyl, or haloalkyl; and
R⁴ is alkyl or alkoxy.

2. The compound of claim 1, having Formula (II): 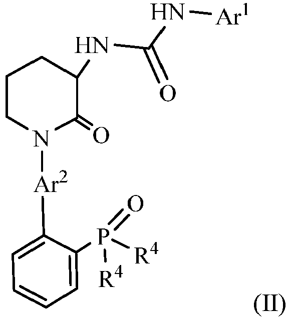 or a pharmaceutically acceptable salt thereof, wherein:
Ar¹ is phenyl substituted with 1 R^{1a} and 1-2 R^{1b} or 6-membered heteroaryl with 1-2 nitrogen atoms and substituted with 1R^{1a} and 1-2 R^{1b};
Ar² is phenyl substituted with 1-3 R² or pyridinyl substituted with 1-3 R²;
R^{1a} is hydrogen or halo;
R^{1b} is halo, C₁₋₄ haloalkyl, C₁₋₄ alkoxy, or C₁₋₄ haloalkoxy;
R² is hydrogen, halo, C₁₋₄ alkyl, C₁₋₄ hydroxyalkyl, C₁₋₄ haloalkyl, C₃₋₆ cycloalkyl, C₁₋₄ alkoxy, or C₁₋₄ haloalkoxy; and
R⁴ is C₁₋₃ alkyl.

3. The compound of claim 1 or 2, having Formula (III): 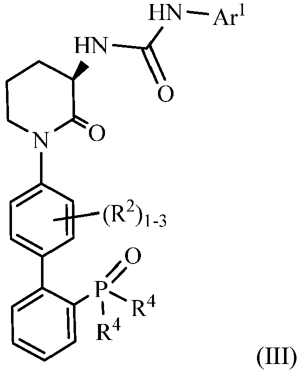 or a pharmaceutically acceptable salt thereof, wherein:
Ar¹ is phenyl substituted with 1R^{1a} and 1-2 R^{1b}, pyridinyl substituted with 1 R^{1a} and 1-2 R^{1b}, or pyrazinyl substituted with 1R^{1a} and 1-2 R^{1b};
R^{1a} is hydrogen or halo;
R^{1b} is halo, C₁₋₄ haloalkyl, C₁₋₄ alkoxy, or C₁₋₄ haloalkoxy;
R² is hydrogen, halo, C₁₋₄ alkyl, C₁₋₄ hydroxyalkyl, C₁₋₄ haloalkyl, C₃₋₆ cycloalkyl, C₁₋₄ alkoxy, or C₁₋₄ haloalkoxy; and
R⁴ is C₁₋₂ alkyl.

4. The compound of claim 3, having Formula (IV): 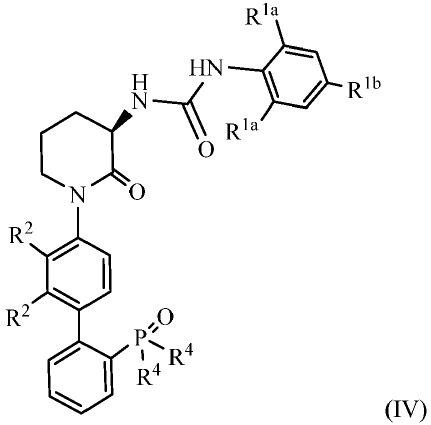 or a pharmaceutically acceptable salt thereof, wherein:
R^{1a} is hydrogen or F;
R^{1b} is halo, C₁₋₂ haloalkyl, or C₁₋₂ alkoxy;
R² is hydrogen, halo, C₁₋₃ alkyl, C₁₋₃ haloalkyl, or C₃₋₆ cycloalkyl; and
R⁴ is methyl or ethyl.

5. The compound of claim 4, having Formula (VI): 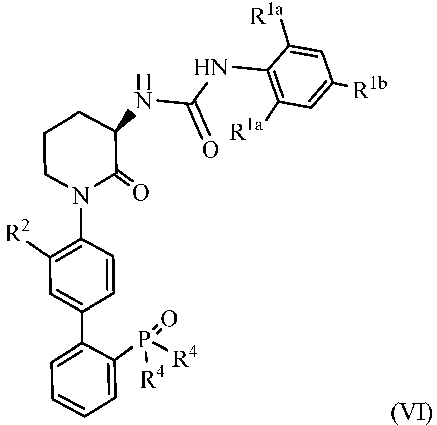 or a pharmaceutically acceptable salt thereof, wherein:
R^{1a} is hydrogen or F;
R^{1b} is halo, C₁₋₂ haloalkyl, or C₁₋₂ alkoxy;
R² is halo, C₁₋₃ alkyl, C₁₋₃ haloalkyl, or C₃₋₆ cycloalkyl; and
R⁴ is methyl or ethyl.

6. The compound of claim 4 or 5, or a pharmaceutically acceptable salt thereof, wherein:
R^{1a} is hydrogen or F;
R^{1b} is F, Cl, or CF₃;
R² is hydrogen, F, Cl, isopropyl, CF₃, or cyclopropyl; and
R⁴ is methyl.

7. The compound of claim 3, having Formula (V): 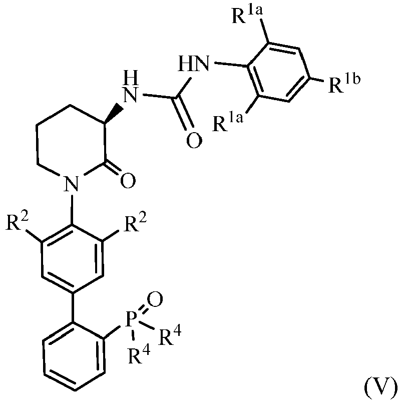 or a pharmaceutically acceptable salt thereof, wherein:
R^{1a} is hydrogen or F;
R^{1b} is halo, C₁₋₂ haloalkyl, or C₁₋₂ alkoxy;
R² is halo; and
R⁴ is methyl or ethyl.

8. The compound of claim 3, having Formula (VII): 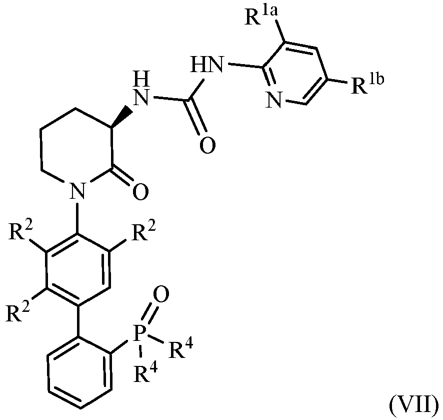 or a pharmaceutically acceptable salt thereof, wherein:
R^{1a} is hydrogen or halo;
R^{1b} is halo, C₁₋₂ haloalkyl, or C₁₋₂ alkoxy;
R² is hydrogen, halo, C₁₋₃ alkyl, or C₃₋₆ cycloalkyl; and
R⁴ is C₁₋₂ alkyl.

9. The compound of claim 3, having Formula (VIII): 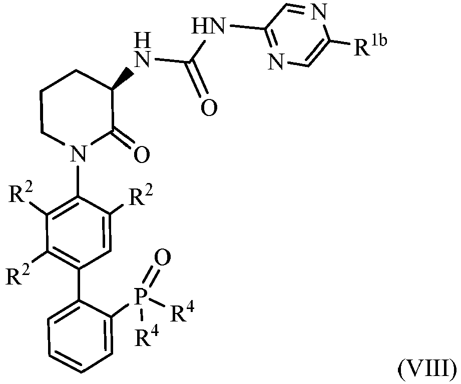 or a pharmaceutically acceptable salt thereof, wherein:
R^{1b} is halo, C₁₋₂ haloalkyl, or C₁₋₂ alkoxy;
R² is hydrogen, halo, C₁₋₃ alkyl, or C₃₋₆ cycloalkyl; and
R⁴ is C₁₋₂ alkyl.

10. The compound of claim 1 or 2, having Formula (IX): 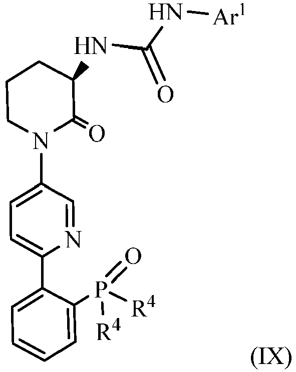 or a pharmaceutically acceptable salt thereof, wherein:
Ar¹ is phenyl substituted with 1 R^{1a} and 1-2 R^{1b}, pyridinyl substituted with 1 R^{1a} and 1-2 R^{1b}, or pyrazinyl substituted with 1R^{1a} and 1-2 R^{1b}.
R^{1a} is hydrogen or halo;
R^{1b} is halo, C₁₋₄ haloalkyl, C₁₋₄ alkoxy, or C₁₋₄ haloalkoxy; and
R⁴ is C₁₋₂ alkyl.

11. A compound of claim 1 selected from the group consisting of: 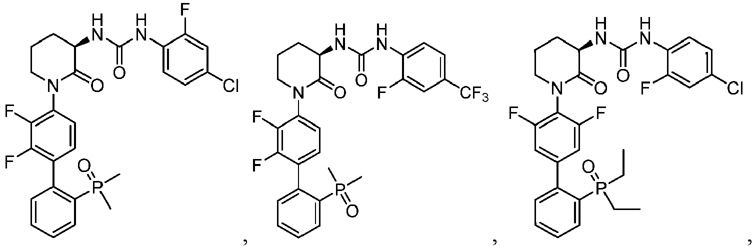 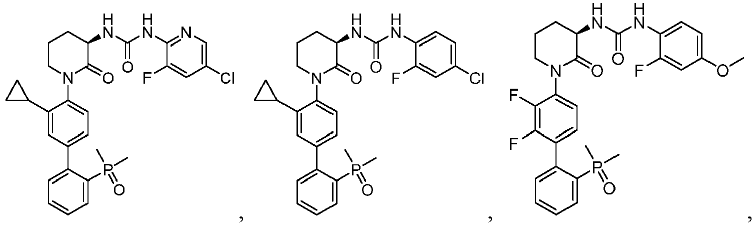 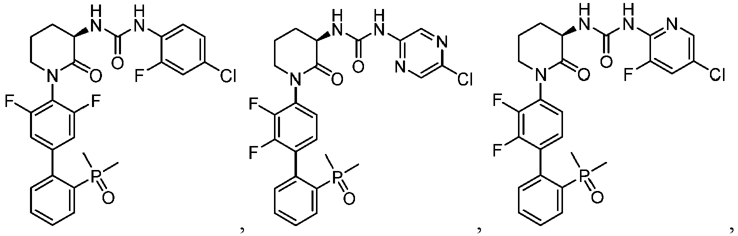 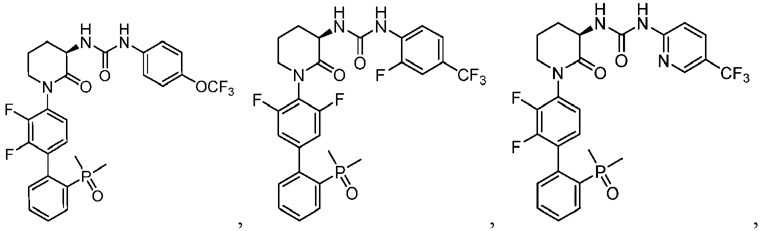 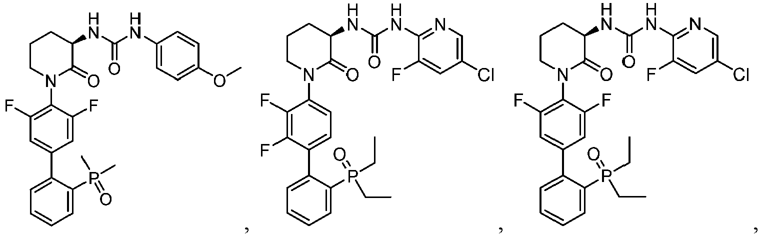 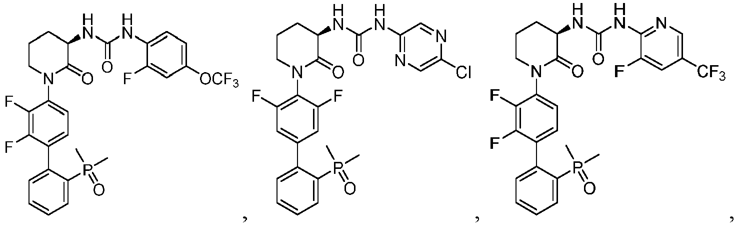 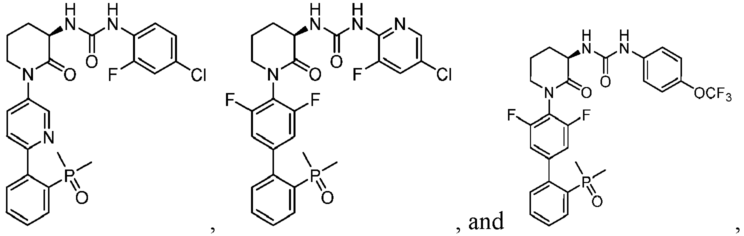 or a pharmaceutically acceptable salt thereof.

12. A composition comprising a compound of any one of claims 1-11, or a pharmaceutically acceptable salt thereof, and a pharmaceutically acceptable carrier, diluent, or excipient.

13. A compound according to any one of claims 1-11 or a pharmaceutically acceptable salt thereof, or composition according to claim 12, for use in therapy.

14. A compound of any of claims 1-11 or a pharmaceutically acceptable salt thereof, or composition according to claim 12, for use in a method for treating a heart disease, the method comprising administering a therapeutically effective amount of a compound of any one of claims 1-11, or a pharmaceutically acceptable salt thereof, to a patient in need thereof.

15. A compound of any claims 1-11 or a pharmaceutically acceptable salt thereof, or composition according to claim 12, for use in treating a heart disease, wherein the heart disease is selected from the group consisting of angina pectoris, unstable angina, myocardial infarction, heart failure, acute coronary disease, and cardiac iatrogenic damage; preferably wherein the heart failure is selected from the group consisting of congestive heart failure, systolic heart failure, diastolic heart failure, heart failure with reduced ejection fraction (HF_{R}EF), heart failure with preserved ejection fraction (HFpEF), acute heart failure, chronic heart failure of ischemic and non-ischemic origin.

## Patentansprüche

1. Verbindung der Formel (I): 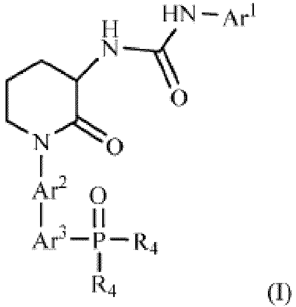 oder ein pharmazeutisch akzeptables Salz davon, wobei:
Ar¹ Phenyl, das mit 1-2 R^{1a} und 1-2 R^{1b} substituiert ist, oder 6-gliedriges Heteroaryl mit 1-3 Stickstoffatomen und mit 1-2 R^{1a} und 1-2 R^{1b} substituiert, ist;
Ar² Phenyl, das mit 1-4 R² substituiert ist, oder 6-gliedriges Heteroaryl mit 1-3 Stickstoffatomen und mit 1-4 R² substituiert, ist;
Ar³ Phenyl, das mit 0-4 R³ substituiert ist, oder Pyridinyl, das mit 0-4 R³ substituiert ist, ist;
R^{1a} Wasserstoff oder Halo ist;
R^{1b} Halo, Haloalkyl, Alkoxy oder Haloalkoxy ist;
R² Wasserstoff, Cyano, Halo, Alkyl, Hydroxyalkyl, Haloalkyl, Cycloalkyl, Alkoxy oder Haloalkoxy ist;
R³ Cyano, Halo, Alkyl, Alkoxy, Hydroxyalkyl,, Alkoxyalkyl oder Haloalkyl ist; und
R⁴ Alkyl oder Alkoxy ist.

2. Verbindung nach Anspruch 1, die die Formel (II) aufweist: 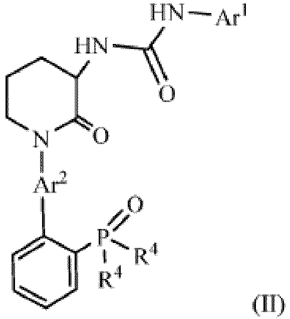 oder ein pharmazeutisch akzeptable Salz davon, wobei:
Ar¹ Phenyl, das mit 1 R^{1a} und 1-2 R^{1b} substituiert ist, oder 6-gliedriges Heteroaryl mit 1-2 Stickstoffatomen und mit 1 R^{1a} und 1-2 R^{1b} substituiert, ist;
Ar² Phenyl, das mit 1-3 R² substituiert ist, oder Pyridinyl, das mit 1-3 R² substituiert, ist;
R^{1a} Wasserstoff oder Halo ist;
R^{1b} Halo, C₁₋₄-Haloalkyl, C₁₋₄-Alkoxy oder C₁₋₄-Haloalkoxy ist;
R² Wasserstoff, Halo, C₁₋₄-Alkyl, C₁₋₄-Hydroxyalkyl, C₁₋₄-Haloalkyl, C₃₋₆-Cycloalkyl, C₁₋₄-Alkoxy oder C₁₋₄-Haloalkoxy ist; und
R⁴ C₁₋₃-Alkyl ist.

3. Verbindung nach Anspruch 1 oder 2, die die Formel (III) aufweist: 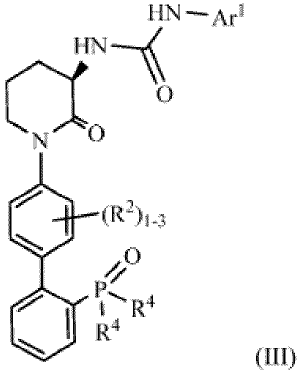 oder ein pharmazeutisch akzeptables Salz davon, wobei:
Ar¹ Phenyl, das mit 1 R^{1a} und 1-2 R^{1b} substituiert ist, Pyridinyl, das mit 1 R^{1a} und 1-2 R^{1b} substituiert ist, oder Pyrazinyl, das mit 1 R^{1a} und 1-2 R^{1b} substituiert ist, ist;
R^{1a} Wasserstoff oder Halo ist;
R^{1b} Halo, C₁₋₄-Haloalkyl, C₁₋₄-Alkoxy oder C₁₋₄-Haloalkoxy ist;
R² Wasserstoff, Halo, C₁₋₄-Alkyl, C₁₋₄-Hydroxyalkyl, C₁₋₄-Haloalkyl, C₃₋₆-Cycloalkyl, C₁₋₄-Alkoxy oder C₁₋₄-Haloalkoxy ist; und
R⁴ C₁₋₂-Alkyl ist.

4. Verbindung nach Anspruch 3, die die Formel (IV) aufweist: 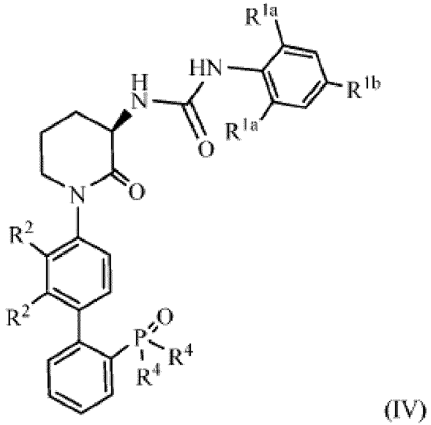 oder ein pharmazeutisch akzeptables Salz davon, wobei:
R^{1a} Wasserstoff oder F ist;
R^{1b} Halo, C₁₋₂-Haloalkyl oder C₁₋₂-Alkoxy ist;
R² Wasserstoff, Halo, C₁₋₃-Alkyl, C₁₋₃-Haloalkyl oder C₃₋₆-Cycloalkyl ist; und
R⁴ Methyl oder Ethyl ist.

5. Verbindung nach Anspruch 4, die die Formel (VI) aufweist: 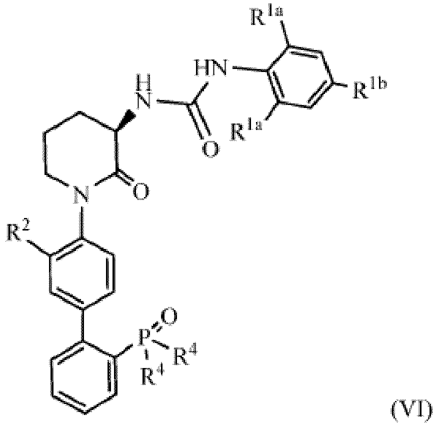 oder ein pharmazeutisch akzeptables Salz davon, wobei:
R^{1a} Wasserstoff oder F ist;
R^{1b} Halo, C₁₋₂-Haloalkyl oder C₁₋₂-Alkoxy ist;
R² Halo, C₁₋₃-Alkyl, C₁₋₃-Haloalkyl oder C₃₋₆-Cycloalkyl ist; und
R⁴ Methyl oder Ethyl ist.

6. Verbindung nach Anspruch 4 oder 5 oder pharmazeutisch akzeptables Salz davon, wobei:
R^{1a} Wasserstoff oder F ist;
R^{1b} F, Cl oder CF₃ ist;
R² Wasserstoff, F, Cl, Isopropyl, CF₃ oder Cyclopropyl ist; und
R⁴ Methyl ist.

7. Verbindung nach Anspruch 3, die die Formel (V) aufweist 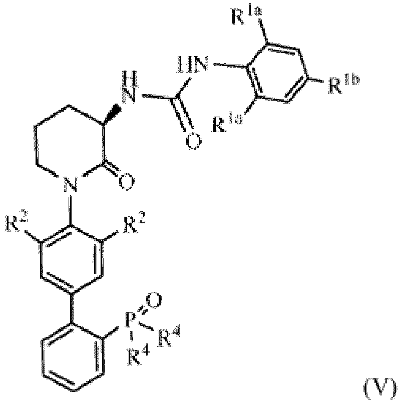 oder ein pharmazeutisch akzeptables Salz davon, wobei:
R^{1a} Wasserstoff oder F ist;
R^{1b} Halo, C₁₋₂-Haloalkyl oder C₁₋₂-Alkoxy ist;
R² Halo ist; und
R⁴ Methyl oder Ethyl ist.

8. Verbindung nach Anspruch 3, die die Formel (VII) aufweist: 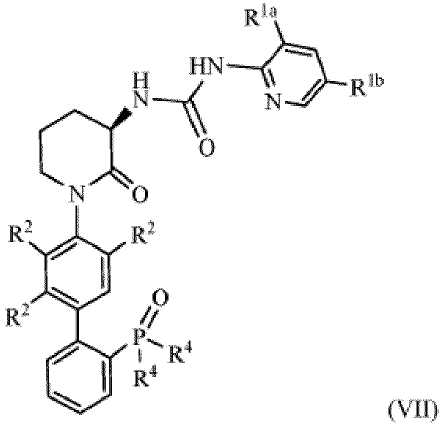 oder ein pharmazeutisch akzeptables Salz davon, wobei:
R^{1a} Wasserstoff oder Halo ist;
R^{1b} Halo, C₁₋₂-Haloalkyl oder C₁₋₂-Alkoxy ist;
R² Wasserstoff, Halo, C₁₋₃-Alkyl oder C₃₋₆-Cycloalkyl ist; und
R⁴ C₁₋₂-Alkyl ist.

9. Verbindung nach Anspruch 3, die die Formel (VIII) aufweist: 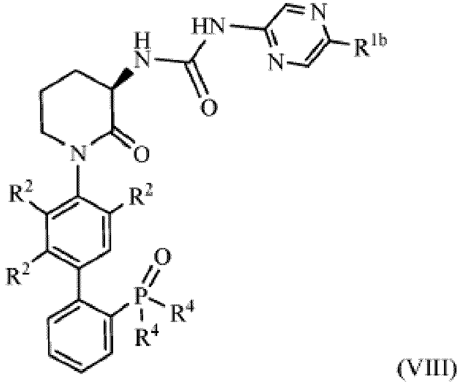 oder ein pharmazeutisch akzeptables Salz davon, wobei:
R^{1b} Halo, C₁₋₂-Haloalkyl oder C₁₋₂-Alkoxy ist;
R² Wasserstoff, Halo, C₁₋₃-Alkyl oder C₃₋₆-Cycloalkyl ist; und
R⁴ C₁₋₂-Alkyl ist.

10. Verbindung nach Anspruch 1 oder 2, die die Formel (IX) aufweist: 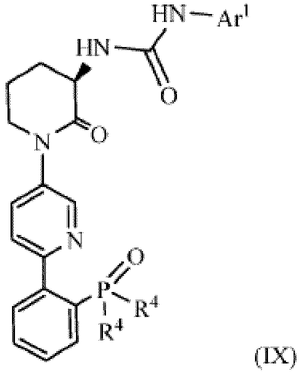 oder ein pharmazeutisch akzeptables Salz davon, wobei:
Ar¹ Phenyl, das mit 1 R^{1a} und 1-2 R^{1b} substituiert ist, Pyridinyl, das mit 1 R^{1a} und 1-2 R^{1b} substituiert ist, oder Pyrazinyl, das mit 1 R^{1a} und 1-2 R^{1b} substituiert ist, ist:
R^{1a} Wasserstoff oder Halo ist;
R^{1b} Halo, C₁₋₄-Haloalkyl, C₁₋₄-Alkoxy oder C₁₋₄-Haloalkoxy ist; und
R⁴ C₁₋₂-Alkyl ist,

11. Verbindung nach Anspruch 1, ausgewählt aus der Gruppe bestehend aus 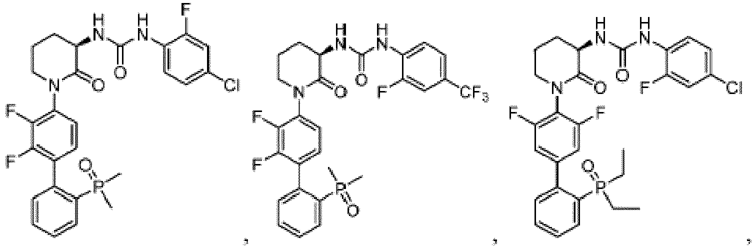 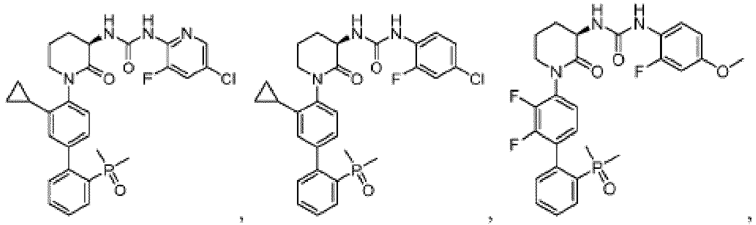 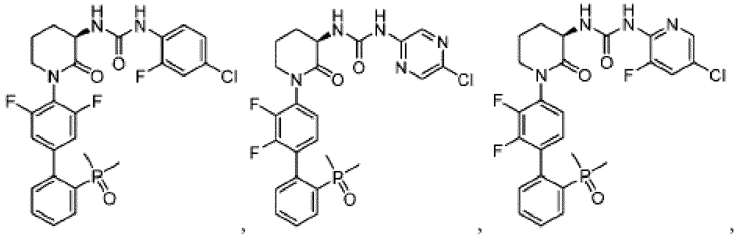 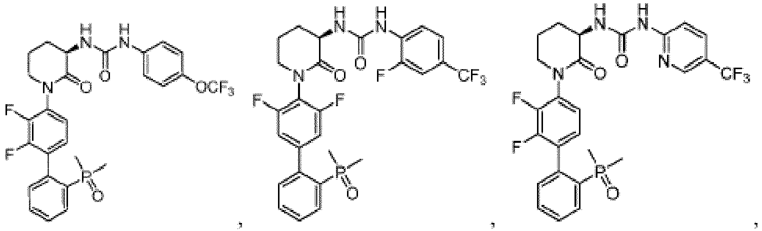 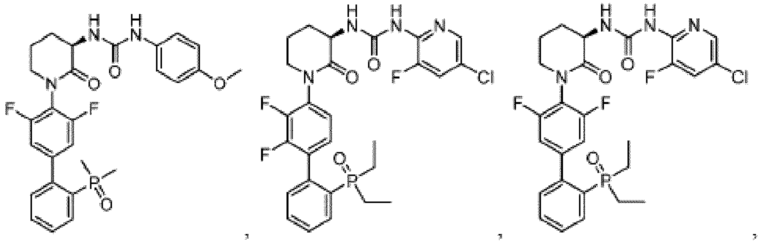 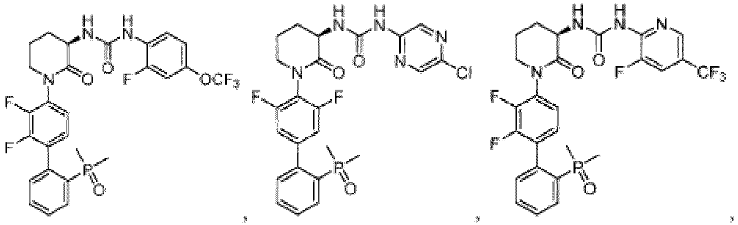 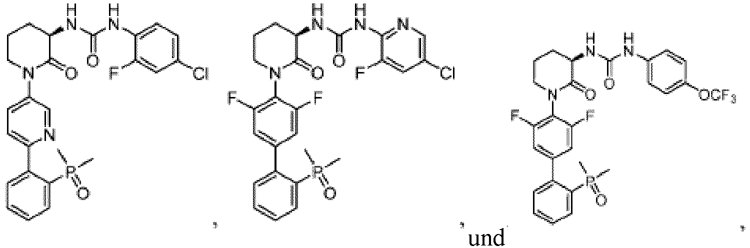 Oder ein pharmazeutisch akzeptables Salz davon.

12. Zusammensetzung umfassend eine Verbindung nach einem der Ansprüche 1-11 oder ein pharmazeutisch akzeptables Salz davon und einen pharmazeutisch akzeptablen Träger, ein pharmazeutisch akzeptables Verdünnungsmittel oder einen pharmazeutisch akzeptablen Trägerstoff.

13. Verbindung nach einem der Ansprüche 1-11 oder ein pharmazeutisch akzeptables Salz davon oder Zusammensetzung nach Anspruch 12 zur Verwendung bei der Therapie.

14. Verbindung nach einem der Ansprüche 1-11 oder ein pharmazeutisch akzeptables Salz davon oder Zusammensetzung nach Anspruch 12 zur Verwendung bei einem Verfahren zum Behandeln einer Herzkrankheit, wobei das Verfahren das Verabreichen einer therapeutisch wirksamen Menge einer Verbindung nach einem der Ansprüche 1-11 oder eines pharmazeutisch akzeptablen Salzes davon einem Patienten, mit Bedarf dafür, umfasst.

15. Verbindung nach einem der Ansprüche 1-11 oder ein pharmazeutisch akzeptables Salz davon oder Zusammensetzung nach Anspruch 12 zur Verwendung zum Behandeln einer Herzkrankheit, wobei die Herzkrankheit aus der Gruppe ausgewählt ist bestehend aus: Angina pectoris, instabiler Angina, Herzmuskelinfarkt, Herzinsuffizienz, akuter Koronarerkrankung, und kardialem iatrogenem Schaden, wobei bevorzugt die Herzinsuffizienz aus der Gruppe ausgewählt wird bestehend aus kongestiver Herzinsuffizient, systolischer Herzinsuffizienz, diastolischer Herzinsuffizienz, Herzinsuffizienz mit reduzierter Auswurffraktion (HF_{R}EF), Herzinsuffizienz mit erhaltener Auswurffraktion (HFₚEF), akuter Herzinsuffizienz, chronischer Herzinsuffizienz ischämischen oder nicht-ischämischen Ursprungs.

## Revendications

1. Composé de la Formule (I) : 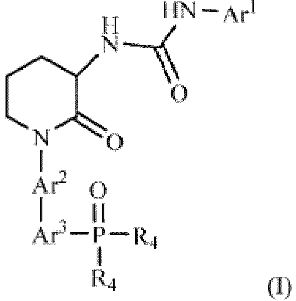 ou un sel pharmaceutiquement acceptable de celui-ci, dans lequel :
Ar¹ est un phényle substitué par 1-2 R^{1a} et 1-2 R^{1b} ou un hétéroaryle à 6 chaînons avec 1-3 atomes d'azote et substitué par 1-2 R^{1a} et 1-2 R^{1b} ;
Ar² est un phényle substitué par 1-4 R² ou un hétéroaryle à 6 chaînons avec 1-3 atomes d'azote et substitué par 1-4 R² ;
Ar³ est un phényle substitué par 0-4 R³ ou un pyridinyle substitué par 0-4 R³ ;
R^{1a} est un hydrogène ou un halo ;
R^{1b} est un halo, un haloalkyle, un alcoxy ou un haloalcoxy ;
R² est un hydrogène, un cyano, un halo, un alkyle, un hydroxyalkyle, un haloalkyle, un cycloalkyle, un alcoxy ou un haloalcoxy ;
R³ est un cyano, un halo, un alkyle, un alcoxy, un hydroxyalkyle, un alcoxyalkyle ou un haloalkyle ; et
R⁴ est un alkyle ou un alcoxy.

2. Composé selon la revendication 1, présentant la Formule (II) : 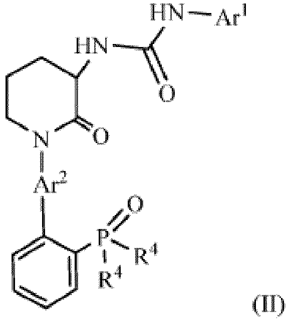 ou un de sel pharmaceutiquement acceptable de celui-ci, dans lequel :
Ar¹ est un phényle substitué par 1 R^{1a} et 1-2 R^{1b} ou un hétéroaryle à 6 chaînons avec 1-2 atomes d'azote et substitué par 1 R^{1a} et 1-2 R^{1b} ;
Ar² est un phényle substitué par 1-3 R² ou un pyridinyle substitué par 1-3 R² ;
R^{1a} est un hydrogène ou un halo ;
R^{1b} est un halo, un C₁₋₄ haloalkyle, un C₁₋₄ alcoxy ou un C₁₋₄ haloalcoxy ;
R² est un hydrogène, un halo, un C₁₋₄ alkyle, un C₁₋₄ hydroxyalkyle, un C₁₋₄ haloalkyle, un C₃₋₆ cycloalkyle, un C₁₋₄ alcoxy ou un C₁₋₄ haloalcoxy ; et
R⁴ est un C₁₋₃ alkyle.

3. Composé selon la revendication 1 ou 2, présentant la Formule (III) : 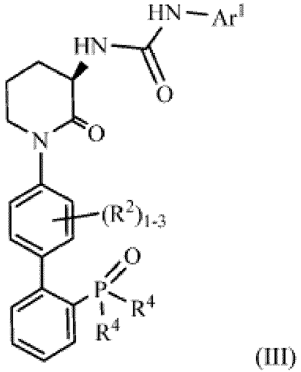 ou un de sel pharmaceutiquement acceptable de celui-ci, dans lequel :
Ar¹ est un phényle substitué par 1 R^{1a} et 1-2 R^{1b}, un pyridinyle substitué par 1 R^{1a} et 1-2 R^{1b} ou un pyrazinyle substitué par 1 R^{1a} et 1-2 R^{1b} ;
R^{1a} est un hydrogène ou un halo ;
R^{1b} est un halo, un C₁₋₄ haloalkyle, un C₁₋₄ alcoxy ou un C₁₋₄ haloalcoxy ;
R² est un hydrogène, un halo, un C₁₋₄ alkyle, un C₁₋₄ hydroxyalkyle, un C₁₋₄ haloalkyle, un C₃₋₆ cycloalkyle, un C₁₋₄ alcoxy ou un C₁₋₄ haloalcoxy ; et
R⁴ est un C₁₋₂ alkyle.

4. Composé selon la revendication 3, présentant la Formule (IV) : 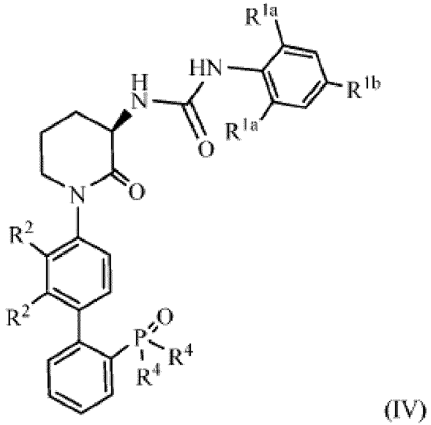 ou un sel pharmaceutiquement acceptable de celui-ci, dans lequel :
R^{1a} est un hydrogène ou F ;
R^{1b} est un halo, un C₁₋₂ haloalkyle ou un C₁₋₂ alcoxy ;
R² est un hydrogène, un halo, un C₁₋₃ alkyle, un C₁₋₃ haloalkyle ou un C₃₋₆ cycloalkyle ; et
R⁴ est un méthyle ou un éthyle.

5. Composé selon la revendication 4, présentant la Formule (VI) : 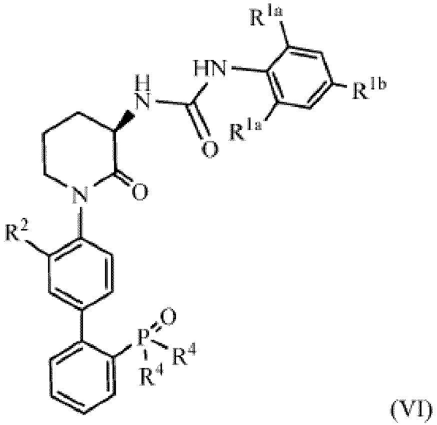 ou un sel pharmaceutiquement acceptable de celui-ci, dans lequel :
R^{1a} est un hydrogène ou F ;
R^{1b} est un halo, un C₁₋₂ haloalkyle ou un C₁₋₂ alcoxy ;
R² est un halo, un C₁₋₃ alkyle, un C₁₋₃ haloalkyle ou un C₃₋₆ cycloalkyle ; et
R⁴ est un méthyle ou un éthyle.

6. Composé selon la revendication 4 ou 5, ou un sel pharmaceutiquement acceptable de celui-ci, dans lequel :
R^{1a} est un hydrogène ou F ;
R^{1b} est F, Cl ou CF₃ ;
R² est un hydrogène, F, Cl, un isopropyle, CF₃ ou un cyclopropyle ; et
R⁴ est un méthyle.

7. Composé selon la revendication 3, présentant la Formule (V) : 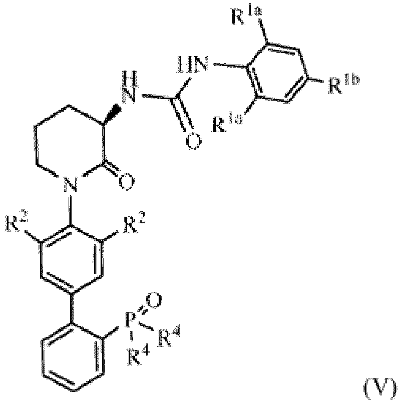 ou un sel pharmaceutiquement acceptable de celui-ci, dans lequel :
R^{1a} est un hydrogène ou F ;
R^{1b} est un halo, un C₁₋₂ haloalkyle ou un C₁₋₂ alcoxy ;
R² est un halo ; et
R⁴ est un méthyle ou un éthyle.

8. Composé selon la revendication 3, présentant la Formule (VII) : 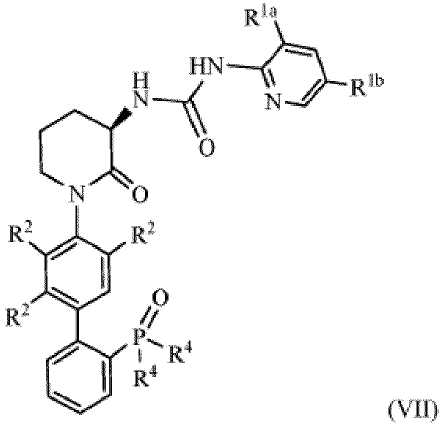 ou un sel pharmaceutiquement acceptable de celui-ci, dans lequel :
R^{1a} est un hydrogène ou un halo ;
R^{1b} est un halo, un C₁₋₂ haloalkyle ou un C₁₋₂ alcoxy ;
R² est un hydrogène, un halo, un C₁₋₃ alkyle ou un C₃₋₆ cycloalkyle ; et
R⁴ est un C₁₋₂ alkyle.

9. Composé selon la revendication 3, présentant la Formule (VIII) : 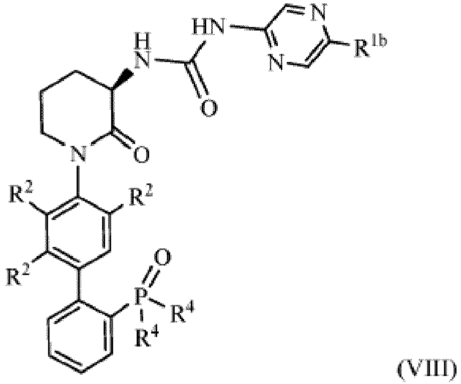 ou un sel pharmaceutiquement acceptable de celui-ci, dans lequel :
R^{1b} est un halo, un C₁₋₂ haloalkyle ou un C₁₋₂ alcoxy ;
R² est un hydrogène, un halo, un C₁₋₃ alkyle ou un C₃₋₆ cycloalkyle ; et
R⁴ est un C₁₋₂ alkyle.

10. Composé selon la revendication 1 ou 2, présentant la Formule (IX) : 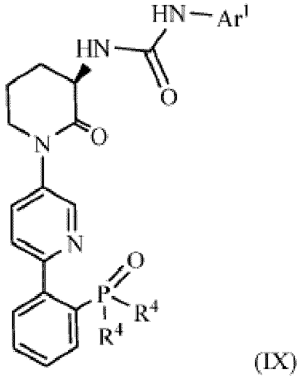 ou un sel pharmaceutiquement acceptable de celui-ci, dans lequel :
Ar¹ est un phényle substitué par 1 R^{1a} et 1-2 R^{1b}, un pyridinyle substitué par 1 R^{1a} et 1-2 R^{1b} ou un pyrazinyle substitué par 1 R^{1a} et 1-2 R^{1b} ;
R^{1a} est un hydrogène ou un halo ;
R^{1b} est un halo, un C₁₋₄ haloalkyle, un C₁₋₄ alcoxy ou un C₁₋₄ haloalcoxy ; et
R⁴ est un C₁₋₂ alkyle.

11. Composé selon la revendication 1 sélectionné dans le groupe constitué de : 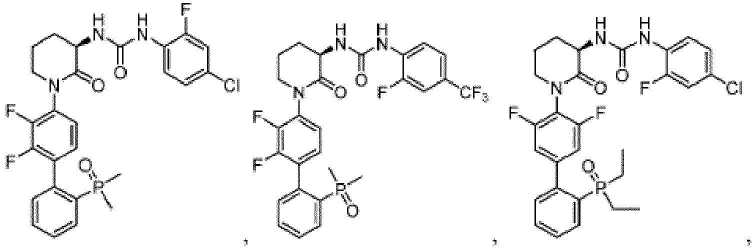 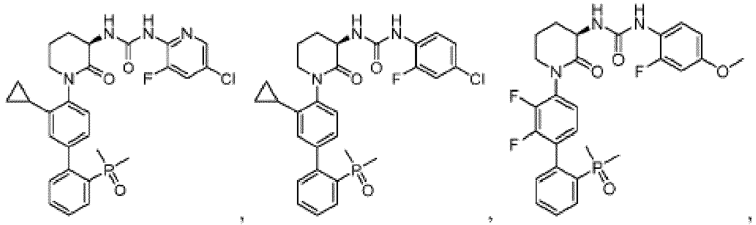 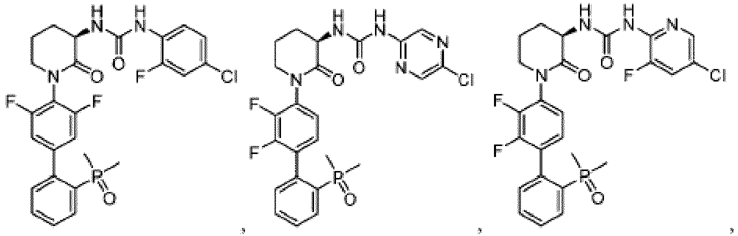 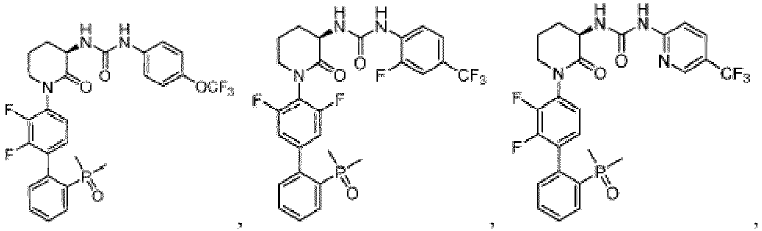 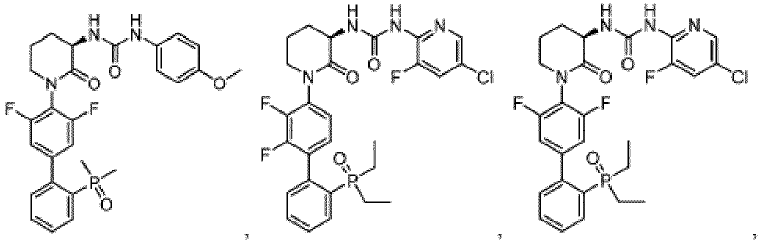 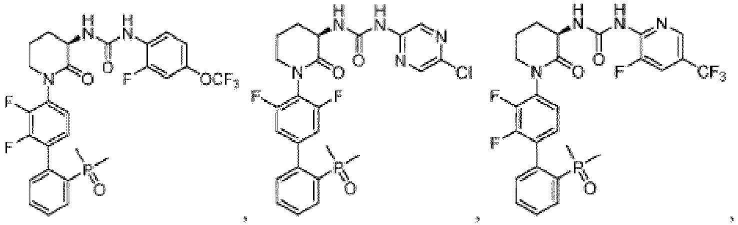 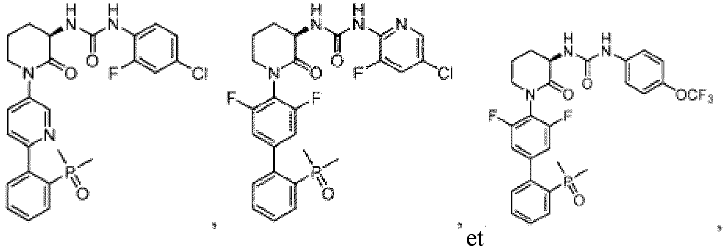 ou un sel pharmaceutiquement acceptable de celui-ci.

12. Composition comprenant un composé selon l'une quelconque des revendications 1-11, ou un sel pharmaceutiquement acceptable de celui-ci, et un véhicule, un diluant ou un excipient pharmaceutiquement acceptables.

13. Composé selon l'une quelconque des revendications 1-11, ou un sel pharmaceutiquement acceptable de celui-ci, ou composition selon la revendication 12, pour utilisation en thérapie.

14. Composé selon l'une quelconque des revendications 1-11, ou un sel pharmaceutiquement acceptable de celui-ci, ou composition selon la revendication 12, pour utilisation dans un procédé pour le traitement d'une maladie cardiaque, le procédé comprenant l'administration d'une quantité thérapeutiquement efficace d'un composé selon l'une quelconque des revendications 1-11, ou d'un sel pharmaceutiquement acceptable de celui-ci, à un patient en ayant besoin.

15. Composé selon l'une quelconque des revendications 1-11, ou un sel pharmaceutiquement acceptable de celui-ci, ou composition selon la revendication 12, pour utilisation dans le traitement d'une maladie cardiaque, où la maladie cardiaque est sélectionnée dans le groupe constitué de : angine de poitrine, angor instable, infarctus du myocarde, insuffisance cardiaque, maladie coronarienne aiguë et dommage iatrogène cardiaque ; de préférence où l'insuffisance cardiaque est sélectionné dans le groupe constitué de : insuffisance cardiaque congestive, insuffisance cardiaque systolique, insuffisance cardiaque diastolique, insuffisance cardiaque avec fraction d'éjection réduite (HF_{R}EF), insuffisance cardiaque avec fraction d'éjection conservée (HFpEF), insuffisance cardiaque aiguë, insuffisance cardiaque chronique d'origines ischémique et non ischémique.
